(19) Europäisches Patentamt
European Patent Office
Office européen des brevets

(11) EP 4 725 947 A1

(12) EUROPEAN PATENT APPLICATION
published in accordance with Art. 153(4) EPC

(43) Date of publication:
15.04.2026 Bulletin 2026/16

(21) Application number: 24818761.9

(22) Date of filing: 07.06.2024

(51) International Patent Classification (IPC):
*C07D 487/04* (2006.01) *A61K 31/517* (2006.01)
*A61P 35/00* (2006.01)

(52) Cooperative Patent Classification (CPC):
A61K 31/517; A61P 35/00; C07D 487/04

(86) International application number:
PCT/CN2024/097937

(87) International publication number:
WO 2024/251230 (12.12.2024 Gazette 2024/50)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 08.06.2023 CN 202310684327

(71) Applicant: Shandong Luye Pharmaceutical Co., Ltd.
Yantai, Shandong 264670 (CN)

(72) Inventors:
- SUN, Jiuchang
  Yantai, Shandong 264670 (CN)
- WANG, Guanghui
  Yantai, Shandong 264670 (CN)
- RUAN, Zheng
  Yantai, Shandong 264670 (CN)

(74) Representative: karo IP
Patentanwälte PartG mbB
Steinstraße 16-18
40212 Düsseldorf (DE)

# (54) SALT FORM AND CRYSTAL FORM OF PLK1 KINASE INHIBITOR, AND PREPARATION METHOD THEREFOR AND USE THEREOF

(57) Provided are a salt form and a crystal form of a PLK1 kinase inhibitor, and a preparation method therefor and the use thereof. Specifically, provided are a salt form and a crystal form of a compound of formula I, a preparation method therefor, and the use thereof in the preparation of a drug for diseases caused by and/or related to PLK1 kinase activity disorders.

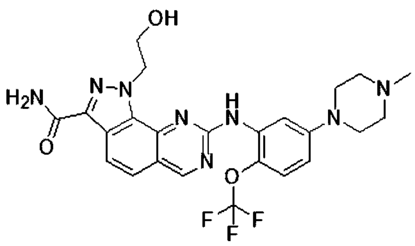

Formula I

EP 4 725 947 A1

## Description

### TECHNICAL FIELD

The present disclosure pertains to the field of pharmaceutical chemistry, and particularly relates to salt forms and crystal forms of a PLK1 kinase inhibitor, a preparation method therefor, and use thereof.

### BACKGROUND

PLK1 is a type of serine/threonine kinase widely present in eukaryotic cells. PLK1 is involved in most processes of cell division, mainly including such biological processes as the entry into cell division, the breakdown of the nuclear membrane, the replication of centrioles, the proper arrangement of chromosomes, the activation of mitotic checkpoints, and cytokinesis.

During the G2 phase, PLK1 plays an important role in the activation of the cyclin B-CDK1 complex through at least two mechanisms. First, it activates the CDC25C phosphatase, which in turn eliminates the inhibitory phosphorylation of CDK1, thereby activating CDK1. Secondly, PLK1 induces the phosphorylation-dependent degradation of WEE1, thereby preventing further phosphorylation of CDK1 and thus allowing cells to enter the M phase. Furthermore, PLK1 is also involved in the regulation of DNA damage checkpoints in the G2 and M phases.

Studies have confirmed that PLK1 is overexpressed in a variety of tumor tissues, and overactive PLK1 expression can enable cells to bypass the DNA damage-induced G2 arrest checkpoint. Furthermore, PLK1 is closely associated with the occurrence and progression of tumors, and the expression level of PLK1 is correlated with poor prognosis in clinical patients. Therefore, PLK1 inhibitors are expected to become effective anti-tumor drug targets.

At present, volasertib and onvansertib are ATP-competitive small-molecule PLK1 inhibitors with relatively rapid progress in clinical trials; whether used as monotherapy or in combination, they have demonstrated significant anti-tumor effects in a variety of preclinical models or clinical studies. Volasertib is an injection, which results in an increase in the incidence of higher-grade adverse events in clinical trials. Onvansertib is an oral preparation, which shows low bioavailability in preclinical studies and has a narrow safety margin in clinical trials. Therefore, there is still a need in the art for compounds with high selectivity, high activity, and better safety to meet clinical needs.

PCT/CN2022/137824 provides a PLK1 kinase inhibitor with a structural formula as shown below. The test results show that this compound has a significant inhibitory effect on PLK1 kinase and can significantly inhibit tumor volume growth. The application is incorporated herein in its entirety.

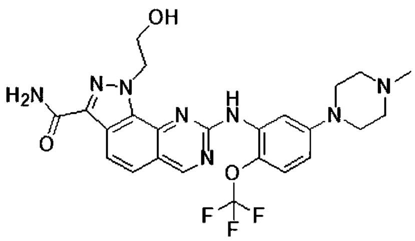

Formula I

The pharmaceutical form (e.g., crystal form or salt) of a compound often affects the chemical stability of the drug. Differences in crystallization conditions and storage conditions may lead to changes in the crystal form structure of the compound that are sometimes accompanied by the production of other crystal forms. Generally, amorphous drug products lack a regular crystal form structure and often have defects such as poor product stability, fine crystallization, difficulty in filtration, proneness to agglomeration, and poor flowability. Given the importance of solid drug salt forms and crystal forms and their stability in clinical treatment, in-depth research on the salt forms and crystal forms of the compound of formula I is of great significance for developing drugs that are suitable for industrial production and have good biological activity.

### SUMMARY

In one aspect, the present disclosure provides a crystal form A of the compound of formula I, having, in an X-ray powder diffraction pattern using Cu-Kα radiation, characteristic diffraction peaks at the following 2θ angles: 4.807±0.2°, 8.532±0.2°, 11.645±0.2°, 15.160±0.2°, 18.347±0.2°, and 25.514±0.2°.

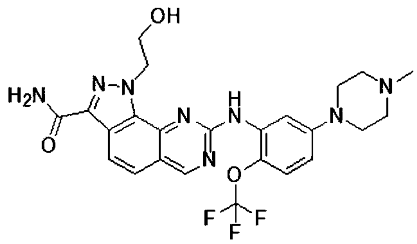

Formula I

In some embodiments, the crystal form A of the compound of formula I has, in an X-ray powder diffraction pattern using Cu-Kα radiation, characteristic diffraction peaks at the following 2θ angles: 4.807±0.2°, 8.532±0.2°, 11.645±0.2°, 15.160±0.2°, 16.768±0.2°, 18.347±0.2°, 19.000±0.2°, 19.247±0.2°, and 25.514±0.2°. In some embodiments, the crystal form A of the compound of formula I has an X-ray powder diffraction pattern using Cu-Kα radiation as shown in FIG. 1-1.

In one aspect, the present disclosure provides a pharmaceutically acceptable salt of the compound of formula I, which is selected from the group consisting of a hydrochloride, sulfate, maleate, L-aspartate, L-tartrate, phosphate, fumarate, citrate, malate, glycolate, L-malate, hippurate, succinate, adipate, p-toluenesulfonate, methanesulfonate, benzenesulfonate, oxalate, malonate, gentisate, or hydrobromide.

In some embodiments, the pharmaceutically acceptable salt is a maleate, fumarate, or succinate.

In some embodiments, in the pharmaceutically acceptable salt, the molar ratio of an acid molecule to the compound of formula I is about 1:2 to 2:1, preferably about 1:1 or 1:2.

In some embodiments, in the pharmaceutically acceptable salt, the molar ratio of maleic acid to the compound of formula I is about 1:1.

In some embodiments, in the pharmaceutically acceptable salt, the molar ratio of fumaric acid to the compound of formula I is about 1:1 or 1:2.

In some embodiments, in the pharmaceutically acceptable salt, the molar ratio of succinic acid to the compound of formula I is about 1:1.

In one aspect, the present disclosure provides a preparation method for the pharmaceutically acceptable salt of the compound of formula I, comprising a step of mixing the compound of formula I with an acid and a solvent. The solvent is one or more solvents selected from the group consisting of ethanol, ethyl acetate, 2-methyltetrahydrofuran, tetrahydrofuran, acetone, water, dichloromethane, dimethyl sulfoxide, N-methylpyrrolidone, acetonitrile, toluene, N,N-dimethylformamide, or n-heptane.

In some embodiments, in the preparation method for the pharmaceutically acceptable salt of the compound of formula I, the molar ratio of the feeding amount of the compound of formula I to the feeding amount of the acid is selected from the group consisting of 5:1 to 1:5, preferably 1:0.5 to 1:1.5, and more preferably 1:1.

In one aspect, the present disclosure provides a crystal form A of a maleate of the compound of formula I, having, in an X-ray powder diffraction pattern using Cu-Kα radiation, characteristic diffraction peaks at the following 20 angles: 8.455±0.2°, 12.802±0.2°, 13.894±0.2°, 19.029±0.2°, 20.917±0.2°, and 21.383±0.2°.

In some embodiments, the crystal form A of the maleate of the compound of formula I has, in an X-ray powder diffraction pattern using Cu-Kα radiation, characteristic diffraction peaks at the following 2θ angles: 8.455±0.2°, 12.313 ±0.2°, 12.802±0.2°, 13.894±0.2°, 17.051±0.2°, 17.713±0.2°, 19.029±0.2°, 20.917±0.2°, 21.383±0.2°, and 24.787 ±0.2°.

In some embodiments, the crystal form A of the maleate of the compound of formula I has, in an X-ray powder diffraction pattern using Cu-Kα radiation, characteristic diffraction peaks at the following 2θ angles: 6.883±0.2°, 8.455 ±0.2°, 12.313±0.2°, 12.802±0.2°, 13.894±0.2°, 14.640±0.2°, 17.051±0.2°, 17.369±0.2°, 17.713±0.2°, 19.029±0.2°, 20.917±0.2°, 21.383±0.2°, 23.818±0.2°, 24.787±0.2°, and 25.823±0.2°.

In some embodiments, the crystal form A of the maleate of the compound of formula I has an X-ray powder diffraction pattern using Cu-Kα radiation as shown in FIG. 2-1.

In some embodiments, in the crystal form A of the maleate of the compound of formula I, the molar ratio of maleic acid to the compound of formula I is about (0.9-1.1):1, preferably 1.0:1.

In an optional embodiment, the present disclosure provides a preparation method for the crystal form A of the maleate of the compound of formula I, comprising steps of mixing the compound of formula I, maleic acid, and a solvent, and crystallizing. In an optional embodiment, the solvent is selected from the group consisting of ethanol, ethyl acetate, 2-methyltetrahydrofuran, or acetone/water. The crystallization method includes anti-solvent addition, gas-solid diffusion, gas-liquid diffusion, stirring at room temperature, stirring with temperature cycling, slow evaporation, or grinding.

In one aspect, the present disclosure provides a crystal form A of a fumarate of the compound of formula I, having,

in an X-ray powder diffraction pattern using Cu-Kα radiation, characteristic diffraction peaks at the following 2θ angles: 7.931±0.2°, 15.752±0.2°, 16.012±0.2°, 20.426±0.2°, 21.636±0.2°, 22.902±0.2°, and 24.234±0.2°.

In some embodiments, the crystal form A of the fumarate of the compound of formula I has, in an X-ray powder diffraction pattern using Cu-Kα radiation, characteristic diffraction peaks at the following 2θ angles: 7.931±0.2°, 12.635 ±0.2°, 13.761±0.2°, 15.752±0.2°, 16.012±0.2°, 16.972±0.2°, 19.481±0.2°, 20.426±0.2°, 21.636±0.2°, 22.285±0.2°, 22.641±0.2°, 22.902±0.2°, 23.597±0.2°, 24.234±0.2°, 27.948±0.2°, and 29.329±0.2°.

In some embodiments, the crystal form A of the fumarate of the compound of formula I has an X-ray powder diffraction pattern using Cu-Kα radiation as shown in FIG. 3-1.

In some embodiments, in the crystal form A of the fumarate of the compound of formula I, the molar ratio of fumaric acid to the compound of formula I is about (0.9-1.1):1, preferably 1.0:1.

In an optional embodiment, the present disclosure provides a preparation method for the crystal form A of the fumarate of the compound of formula I, comprising steps of mixing the compound of formula I, fumaric acid, and a solvent (I), and crystallizing. In an optional embodiment, the solvent (I) is selected from the group consisting of ethanol, ethyl acetate, 2-methyltetrahydrofuran, acetone/water, N-methylpyrrolidone, N,N-dimethylformamide, or tetrahydrofuran/-water. The crystallization method includes anti-solvent addition, gas-solid diffusion, gas-liquid diffusion, stirring at room temperature, stirring with temperature cycling, slow evaporation, or grinding. The anti-solvent is selected from the group consisting of methanol, isopropanol, methyl isobutyl ketone, isopropyl acetate, toluene, 2-butanol, butanone, ethyl acetate, or trichloromethane.

In one aspect, the present disclosure provides a crystal form A of a succinate of the compound of formula I, having, in an X-ray powder diffraction pattern using Cu-Kα radiation, characteristic diffraction peaks at the following 2θ angles: 7.771±0.2°, 13.539±0.2°, 15.519±0.2°, 20.105±0.2°, 21.347±0.2°, and 22.552±0.2°.

In some embodiments, the crystal form A of the succinate of the compound of formula I has, in an X-ray powder diffraction pattern using Cu-Kα radiation, characteristic diffraction peaks at the following 2θ angles: 6.655±0.2°, 7.771 ±0.2°, 13.539±0.2°, 15.519±0.2°, 15.781±0.2°, 17.709±0.2°, 20.105±0.2°, 21.347±0.2°, 22.552±0.2°, and 23.887 ±0.2°.

In some embodiments, the crystal form A of the succinate of the compound of formula I has an X-ray powder diffraction pattern using Cu-Kα radiation as shown in FIG. 4-1.

In some embodiments, in the crystal form A of the succinate of the compound of formula I, the molar ratio of succinic acid to the compound of formula I is about (0.9-1.1):1, preferably 1.0:1.

In an optional embodiment, the present disclosure provides a preparation method for the crystal form A of the succinate of the compound of formula I, comprising steps of mixing the compound of formula I, succinic acid, and a solvent, and crystallizing. In an optional embodiment, the solvent is selected from the group consisting of ethanol, ethyl acetate, 2-methyltetrahydrofuran, or acetone/water. The crystallization method includes anti-solvent addition, gas-solid diffusion, gas-liquid diffusion, stirring at room temperature, stirring with temperature cycling, slow evaporation, or grinding.

In one aspect, the present disclosure provides a crystal form B of a hemifumarate of the compound of formula I, having, in an X-ray powder diffraction pattern using Cu-Kα radiation, characteristic diffraction peaks at the following 2θ angles: 15.084±0.2°, 15.501±0.2°, 17.311±0.2°, 24.838±0.2°, 26.132±0.2°, and 26.736±0.2°.

In some embodiments, the crystal form B of the hemifumarate of the compound of formula I has, in an X-ray powder diffraction pattern using Cu-Kα radiation, characteristic diffraction peaks at the following 2θ angles: 8.570±0.2°, 12.545±0.2°, 15.084±0.2°, 15.501±0.2°, 17.311±0.2°, 24.838±0.2°, 26.132±0.2°, and 26.736±0.2°.

In some embodiments, the crystal form B of the hemifumarate of the compound of formula I has an X-ray powder diffraction pattern using Cu-Kα radiation as shown in FIG. 5-1.

In some embodiments, in the crystal form B of the hemifumarate of the compound of formula I, the molar ratio of fumaric acid to the compound of formula I is about (0.5-0.7):1, preferably (0.6-0.7):1.

In some embodiments, the crystal form B of the hemifumarate of the compound of formula I is an NMP solvate; preferably, the molar ratio of NMP (i.e., N-methylpyrrolidone) to the compound of formula I is about (0.9-2.0): 1, or the molar ratio of NMP to the compound of formula I is about 1.1:1 or 2:1.

In an optional embodiment, the present disclosure provides a preparation method for the crystal form B of the hemifumarate of the compound of formula I, comprising mixing the compound of formula I, fumaric acid, and a solvent (I), and adding an anti-solvent under stirring conditions. In an optional embodiment, the solvent (I) is N-methylpyrrolidone, and the anti-solvent is methyl tert-butyl ether. The crystallization method includes anti-solvent addition, gas-solid diffusion, gas-liquid diffusion, stirring at room temperature, stirring with temperature cycling, slow evaporation, or grinding.

In one aspect, the present disclosure provides a crystal form C of a hemifumarate of the compound of formula I, having, in an X-ray powder diffraction pattern using Cu-Kα radiation, characteristic diffraction peaks at the following 2θ angles: 7.553±0.2°, 12.716±0.2°, 13.547±0.2°, 15.313±0.2°, 19.089±0.2°, and 22.755±0.2°.

In some embodiments, the crystal form C of the hemifumarate of the compound of formula I has, in an X-ray powder diffraction pattern using Cu-Kα radiation, characteristic diffraction peaks at the following 2θ angles: 6.637±0.2°, 7.553±0.2°, 12.716±0.2°, 13.547±0.2°, 15.313±0.2°, 19.089±0.2°, 20.121±0.2°, 20.609±0.2°, and 22.755±0.2°.

In some embodiments, the crystal form C of the hemifumarate of the compound of formula I has an X-ray powder diffraction pattern using Cu-Kα radiation as shown in FIG. 6-1.

In some embodiments, in the crystal form C of the hemifumarate of the compound of formula I, the molar ratio of fumaric acid to the compound of formula I is about (0.5-0.7):1, preferably 0.5:1.

In some embodiments, the crystal form C of the hemifumarate of the compound of formula I is a hydrate.

In some embodiments, the present disclosure provides a preparation method for the crystal form C of the hemifumarate of the compound of formula I, comprising steps of mixing the compound of formula I, fumaric acid, and a solvent (I), and crystallizing. In an optional embodiment, the solvent (I) is selected from the group consisting of N-methylpyrrolidone, N,N-dimethylformamide, acetone/water, methanol/water, tetrahydrofuran/water, isopropanol/water, or acetonitrile/water. The crystallization method includes anti-solvent addition, gas-solid diffusion, gas-liquid diffusion, stirring at room temperature, stirring with temperature cycling, slow evaporation, or grinding.

In some embodiments, the present disclosure provides a preparation method for the crystal form C of the hemifumarate of the compound of formula I, comprising mixing the compound of formula I, fumaric acid, and a solvent (I), wherein the solvent (I) is selected from the group consisting of N-methylpyrrolidone or N,N-dimethylformamide; and adding an anti-solvent, wherein the anti-solvent is selected from the group consisting of water or acetone.

In some embodiments, the present disclosure provides a preparation method for the crystal form C of the hemifumarate of the compound of formula I, comprising adding the crystal form A of the fumarate of the compound of formula I to a solvent, then adding a PVP/PVC polymer, and slowly evaporating at room temperature. The solvent is selected from the group consisting of acetone/water, methanol/water, or tetrahydrofuran/water.

In one aspect, the present disclosure provides a crystal form D of a hemifumarate of the compound of formula I, having, in an X-ray powder diffraction pattern using Cu-Kα radiation, characteristic diffraction peaks at the following 2θ angles: 6.881±0.2°, 8.103±0.2°, 12.572±0.2°, and 13.831±0.2°.

In some embodiments, the crystal form D of the hemifumarate of the compound of formula I has, in an X-ray powder diffraction pattern using Cu-Kα radiation, characteristic diffraction peaks at the following 2θ angles: 6.881±0.2°, 8.103±0.2°, 12.572±0.2°, 13.831±0.2°, 19.569±0.2°, 20.533±0.2°, and 24.021±0.2°.

In some embodiments, the crystal form D of the hemifumarate of the compound of formula I has an X-ray powder diffraction pattern using Cu-Kα radiation as shown in FIG. 7-1.

In some embodiments, in the crystal form D of the hemifumarate of the compound of formula I, the molar ratio of fumaric acid to the compound of formula I is about (0.5-0.7):1, preferably 0.5:1.

In some embodiments, the crystal form D of the hemifumarate of the compound of formula I is a channel hydrate or an anhydrate.

In some embodiments, the present disclosure provides a preparation method for the crystal form D of the hemifumarate of the compound of formula I, comprising heating the crystal form C of the hemifumarate of the compound of formula I to 140 °C to give the crystal form D.

In some embodiments, the present disclosure provides a preparation method for the crystal form D of the hemifumarate of the compound of formula I, comprising mixing the compound of formula I, fumaric acid, and tetrahydrofuran/water, adding acetone as an anti-solvent, and cooling to 5 °C to give the crystal form D.

In one aspect, the present disclosure provides a crystal form E of a hemifumarate of the compound of formula I, having, in an X-ray powder diffraction pattern using Cu-Kα radiation, characteristic diffraction peaks at the following 2θ angles: 6.188±0.2°, 12.492±0.2°, 15.473±0.2°, 17.729±0.2°, 25.017±0.2°, and 26.603±0.2°.

In some embodiments, the crystal form E of the hemifumarate of the compound of formula I has, in an X-ray powder diffraction pattern using Cu-Kα radiation, characteristic diffraction peaks at the following 2θ angles: 6.188±0.2°, 12.492±0.2°, 15.473±0.2°, 17.729±0.2°, 25.017±0.2°, 25.275±0.2°, 26.603±0.2°, and 31.795±0.2°. In some embodiments, the crystal form E of the hemifumarate of the compound of formula I has an X-ray powder diffraction pattern using Cu-Kα radiation as shown in FIG. 8-1.

In some embodiments, in the crystal form E of the hemifumarate of the compound of formula I, the molar ratio of fumaric acid to the compound of formula I is about (0.5-0.7):1, preferably 0.7:1.

In some embodiments, the crystal form E of the hemifumarate of the compound of formula I is a DMAc/$CHCl_3$ co-solvate, wherein the molar ratio of DMAc (i.e., N,N-dimethylacetamide) to the compound of formula I is about 0.4:1, and the molar ratio of $CHCl_3$ to the compound of formula I is about 0.3:1.

The present disclosure provides a preparation method for the crystal form E of the hemifumarate of the compound of formula I, comprising adding the crystal form A of the fumarate of the compound of formula I to N,N-dimethylacetamide for complete dissolution, adding trichloromethane as an anti-solvent, sealing the mixture, and placing the mixture at room temperature until a solid is precipitated.

In one aspect, the present disclosure provides a crystal form F of a hemifumarate of the compound of formula I, having, in an X-ray powder diffraction pattern using Cu-Kα radiation, characteristic diffraction peaks at the following 2θ angles: 8.866±0.2°, 16.651±0.2°, 25.199±0.2°, and 26.157±0.2°.

In some embodiments, the crystal form F of the hemifumarate of the compound of formula I has, in an X-ray

powder diffraction pattern using Cu-Kα radiation, characteristic diffraction peaks at the following 2θ angles: 8.185±0.2°, 8.866±0.2°, 13.449±0.2°, 16.651±0.2°, 16.841±0.2°, 25.199±0.2°, and 26.157±0.2°.

In some embodiments, the crystal form F of the hemifumarate of the compound of formula I has an X-ray powder diffraction pattern using Cu-Kα radiation as shown in FIG. 9-1.

In some embodiments, in the crystal form F of the hemifumarate of the compound of formula I, the molar ratio of fumaric acid to the compound of formula I is about (0.5-0.7):1, preferably 0.5:1 or 0.6:1.

In some embodiments, the crystal form F of the hemifumarate of the compound of formula I is a DMAc (i.e., N,N-dimethylacetamide) solvate, wherein the molar ratio of DMAc to the compound of formula I is about 0.5:1. In some embodiments, the present disclosure provides a preparation method for the crystal form F of the hemifumarate of the compound of formula I, comprising adding the crystal form A of the fumarate of the compound of formula I to N,N-dimethylacetamide for complete dissolution, adding an anti-solvent selected from the group consisting of methanol, butanone, or methyl tert-butyl ether, sealing the mixture, and placing the mixture at room temperature until a solid is precipitated.

In one aspect, the present disclosure provides a crystal form G of a hemifumarate of the compound of formula I, having, in an X-ray powder diffraction pattern using Cu-Kα radiation, characteristic diffraction peaks at the following 2θ angles: 8.925±0.2°, 12.025±0.2°, 12.948±0.2°, 13.525±0.2°, 15.763±0.2°, 16.793±0.2°, 21.133±0.2°, 23.547±0.2°, 25.164±0.2°, and 26.101±0.2°.

In some embodiments, the crystal form G of the hemifumarate of the compound of formula I has, in an X-ray powder diffraction pattern using Cu-Kα radiation, characteristic diffraction peaks at the following 2θ angles: 6.707±0.2°, 7.294±0.2°, 8.925±0.2°, 12.025±0.2°, 12.948±0.2°, 13.525±0.2°, 15.510±0.2°, 15.763±0.2°, 16.417±0.2°, 16.793 ±0.2°, 18.923±0.2°, 21.133±0.2°, 23.547±0.2°, 25.164±0.2°, and 26.101±0.2°.

In some embodiments, the crystal form G of the hemifumarate of the compound of formula I has an X-ray powder diffraction pattern using Cu-Kα radiation as shown in FIG. 10-1.

In some embodiments, in the crystal form G of the hemifumarate of the compound of formula I, the molar ratio of fumaric acid to the compound of formula I is about (0.5-0.7):1, preferably 0.6:1.

In some embodiments, the crystal form G of the hemifumarate of the compound of formula I is an NMP solvate, wherein the molar ratio of NMP to the compound of formula I is about 0.5:1.

In some embodiments, provided is a preparation method for the crystal form G of the hemifumarate of the compound of formula I, comprising heating the crystal form B of the hemifumarate of the compound of formula I to about 140 °C.

In an optional embodiment, in the preparation method for any one of the crystal forms described above, the crystallization method is one or more crystallization methods selected from the group consisting of crystallization at room temperature, gas-liquid diffusion crystallization, gas-solid diffusion crystallization, polymer-induced crystallization, cooling crystallization, solvent evaporation crystallization, anti-solvent addition crystallization, or seed crystal addition-induced crystallization.

The present disclosure further provides a pharmaceutical composition, comprising the crystal form A of the compound of formula I, any one of the aforementioned pharmaceutically acceptable salts or crystal forms thereof, and a pharmaceutically acceptable carrier. The pharmaceutical composition may be prepared into various pharmaceutically acceptable dosage forms, such as tablets, capsules, oral liquids, granules, injections, or various sustained-release and controlled-release preparations. The pharmaceutical composition may be administered orally or parenterally (e.g., intravenously, subcutaneously, or topically). The administration dose may be appropriately adjusted based on the patient's age, sex, and disease type, and is generally about 1-200 mg per day. In one aspect, the present disclosure further provides use of the crystal form A of the compound of formula I, any one of the aforementioned pharmaceutically acceptable salts or crystal forms, or the pharmaceutical composition in the preparation of a medicament for treating a disease caused by and/or associated with dysregulation of PLK1 kinase activity.

In one aspect, the present disclosure further provides use of the crystal form A of the compound of formula I, any one of the aforementioned pharmaceutically acceptable salts or crystal forms, or the pharmaceutical composition in the preparation of a medicament for preventing and/or treating a disease caused by and/or associated with dysregulation of protein kinase activity. The disease includes a cancer, a cell proliferative disease, a viral infection, an autoimmune disease, and a neurodegenerative disease.

The cancer includes, but is not limited to: carcinomas such as those of the bladder, breast, colon, kidney, liver, lung (including small cell lung cancer), esophagus, gallbladder, ovary, pancreas, stomach, cervix, thyroid, prostate, and skin (including squamous cell carcinoma); hematopoietic tumors of lymphoid lineage, including leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma, and Burkitt's lymphoma; hematopoietic tumors of myeloid lineage, including acute and chronic myelogenous leukemias, myelodysplastic syndrome, and promyelocytic leukemia; tumors of mesenchymal origin, including fibrosarcoma and rhabdomyosarcoma; tumors of the central and peripheral nervous systems, including astrocytoma, neuroblastoma, glioma, and schwannoma; and other tumors, including melanoma, seminoma,

teratoma, osteosarcoma, xeroderma pigmentosum, keratoacanthoma, thyroid follicular carcinoma, and Kaposi's sarcoma.

The cell proliferative disease includes, for example, benign prostatic hyperplasia, familial adenomatosis, polyposis, neurofibromatosis, psoriasis,
vascular smooth cell proliferation associated with atherosclerosis, pulmonary fibrosis, arthritis, glomerulonephritis, and postoperative stenosis and restenosis.

**Definitions and Explanations**

Unless otherwise specified, the following terms and phrases used herein are intended to have the following meanings. A particular term or phrase, when not specifically defined, should not be considered indefinite or unclear, but should be construed according to its common meaning. When referring to a trade name, it is intended to refer to its corresponding commercial product or its active ingredient.

The term "pharmaceutically acceptable" is described in the present disclosure in terms of the compounds, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The compounds of the present disclosure may have asymmetric carbon atoms (optical centers) or double bonds. Racemates, diastereoisomers, geometric isomers, and individual isomers are all included within the scope of the present disclosure.

The compounds of the present disclosure may be present in particular geometric or stereoisomeric forms. All such compounds are contemplated herein, including cis and trans isomers, (-)- and (+)-enantiomers, (R)- and (S)-enantiomers, diastereoisomers, (D)-isomers, (L)-isomers, and racemic mixtures and other mixtures thereof, such as enantiomerically or diastereoisomerically enriched mixtures, all of which fall within the scope of the present disclosure. Additional asymmetric carbon atoms may be present in substituents such as alkyl. All such isomers and mixtures thereof are included within the scope of the present disclosure.

The term "pharmaceutically acceptable carrier" refers to any preparation or carrier medium that is capable of delivering an effective amount of the active substance of the present disclosure, does not interfere with the biological activity of the active substance, and has no toxic or side effects on a host or patient; representative carriers include, but are not limited to: binders, fillers, lubricants, disintegrants, wetting agents, dispersing agents, solubilizers, suspending agents, etc.

The present disclosure is intended to include all isotopes of atoms present in the compounds of the present disclosure. Isotopes include those atoms having the same atomic number but different mass numbers. As a general and non-limiting example, isotopes of hydrogen include deuterium and tritium. Isotopes of carbon include $^{13}C$ and $^{14}C$. Isotopically labeled compounds of the present disclosure can generally be prepared by conventional techniques known to those skilled in the art or by methods similar to those described herein using an appropriate isotopically labeled reagent in place of the non-labeled reagent otherwise used.

**BRIEF DESCRIPTION OF THE DRAWINGS**

FIGs. 1-1, 1-2, and 1-3 show an XRPD pattern, TGA/DSC thermograms, and a $^{1}H$ NMR spectrum of the crystal form A of the compound of formula I, respectively.

FIGs. 2-1, 2-2, and 2-3 show an XRPD pattern, a $^{1}H$ NMR spectrum, and TGA/DSC thermograms of the crystal form A of the maleate of the compound of formula I, respectively.

FIGs. 3-1, 3-2, and 3-3 show an XRPD pattern, a $^{1}H$ NMR spectrum, and TGA/DSC thermograms of the crystal form A of the fumarate of the compound of formula I, respectively.

FIGs. 4-1, 4-2, and 4-3 show an XRPD pattern, a $^{1}H$ NMR spectrum, and TGA/DSC thermograms of the crystal form A of the succinate of the compound of formula I, respectively.

FIGs. 5-1, 5-2, and 5-3 show an XRPD pattern, a $^{1}H$ NMR spectrum, and TGA/DSC thermograms of the crystal form B of the hemifumarate of the compound of formula I, respectively.

FIGs. 6-1, 6-2, and 6-3 show an XRPD pattern, a $^{1}H$ NMR spectrum, and TGA/DSC thermograms of the crystal form C of the hemifumarate of the compound of formula I, respectively.

FIGs. 7-1, 7-2, and 7-3 show an XRPD pattern, a $^{1}H$ NMR spectrum, and TGA/DSC thermograms of the crystal form D of the hemifumarate of the compound of formula I, respectively.

FIGs. 8-1, 8-2, and 8-3 show an XRPD pattern, a $^{1}H$ NMR spectrum, and TGA/DSC thermograms of the crystal form E of the hemifumarate of the compound of formula I, respectively.

FIG. 8-4 shows XRPD patterns of the crystal form E of the hemifumarate of the compound of formula I before and after heating.

FIGs. 9-1, 9-2, and 9-3 show an XRPD pattern, a $^1H$ NMR spectrum, and TGA/DSC thermograms of the crystal form F of the hemifumarate of the compound of formula I, respectively.

FIGs. 10-1, 10-2, and 10-3 show an XRPD pattern, a $^1H$ NMR spectrum, and TGA/DSC thermograms of the crystal form G of the hemifumarate of the compound of formula I, respectively.

FIG. 11 shows animal tumor growth curves in an *in vivo* pharmacodynamic study of the compound of formula I in mice.

FIG. 12 shows body weights of animals during the *in vivo* pharmacodynamic study of the compound of formula I in mice.

DETAILED DESCRIPTION

The present disclosure is further illustrated below with reference to specific examples and test examples, but these examples do not limit the scope of the present disclosure in any way.

Test conditions for the instruments used in the experiments:

**X-ray powder diffraction (XRPD)**

(1) Except for the crystal form D of the hemifumarate of the compound of formula I, the X-ray powder diffraction data for all other crystal forms were collected using a Bruker D2 X-ray powder diffractometer under ambient conditions. A sample (about 2 mg) was taken, spread on a high-purity silicon sample stage, flattened using a glass slide with a weighing paper in between, and loaded for analysis. The X-ray tube used a Cu target (Kα), with a Kα2/Kα1 intensity ratio of 0.50 (1.54439 Å/1.5406 Å). The X-ray emitter had a power of 300 W, a voltage of 30 kV, and a current of 10 mA. The divergence slit was 0.6 mm, and the Soller slit was 4.0°. The step rate was 0.15 s/step, the step size was 0.02° (2θ), and the total number of steps was 1837.

(2) Rigaku Smart SE: A Rigaku X-ray powder diffractometer was used, with a voltage of 40 kV and a current of 40 mA. The X-ray tube used a Cu target (Kα), with a Kα2/Kα1 intensity ratio of 0.50 (1.544414 Å/1.540593 Å). The incident slit was 0.5°, and the receiving slit was 20.0 mm. A sample (1-2 mg) was collected under ambient conditions and flattened on a sample stage with no background signal to prepare a test sample, and then X-ray powder diffraction data of the test sample were collected under ambient conditions. The test range was 3-40° (2θ), the step rate was 8 °/min, and the step size was 0.01°/step.

**Thermogravimetric analyzer (TGA)**

Thermogravimetric data for samples were collected using a TA Discovery-series thermogravimetric analyzer (TGA550). A sample (several milligrams) was taken, placed in a Tzero aluminum pan (the sample was automatically weighed by the instrument in the test), and heated from room temperature to the target temperature in a $N_2$ atmosphere, with a $N_2$ flow rate of 60 mL/min and a ramp rate of 10 °C/min.

**Differential scanning calorimeter (DSC)**

Thermal data for samples were collected using a TA Discovery-series differential scanning calorimeter (DSC2500). A sample (several milligrams) was weighed into a Tzero aluminum pan, and the pan was sealed with a Tzero hermetic lid. The sample was heated to the target temperature (before the decomposition temperature) in a $N_2$ atmosphere, with a $N_2$ flow rate of 50 mL/min and a ramp rate of 10 °C/min.

**Proton nuclear magnetic resonance spectroscopy ($^1$H NMR)**

Samples were dissolved in DMSO-$d_6$ to form solutions with a concentration of about 2-10 mg/mL, and proton nuclear magnetic resonance spectroscopic data for the samples were collected using Bruker AVANCE NEO 400 MHZ.

**High performance liquid chromatography (HPLC)**

The purity of samples was determined using an Agilent 1260 high performance liquid chromatograph (equipped with a DAD detector) with a Zorbax Eclipse XDB-C18 column (4.6 × 250 mm, 5 μm).

**Example 1. Synthesis and Characterization of Compound of Formula I**

1.1. Synthesis of intermediate **1-2**

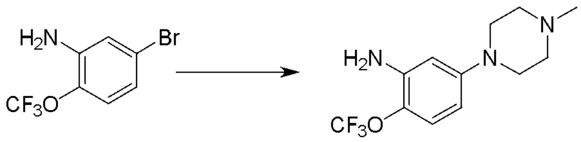

**1-1** **1-2**

Intermediate **1-1** (20.0 g, 78.1 mmol), bis(dibenzylideneacetone)palladium (0.715 g, 0.78 mmol), and 2-dicyclohexylphosphino-2'-(N,N-dimethylamino)-biphenyl (0.615 g, 1.56 mmol) were dissolved in tetrahydrofuran (32 mL), and N-methylpiperazine (12.5 g, 125 mmol) and lithium bis(trimethylsilyl)amide (1.00 M, 187 mL) were added at 0 °C. The mixture was allowed to react at 70 °C for 3 h. Water (200 mL) was added to quench the reaction, and then dichloromethane (100 mL × 3) was added for extraction. The organic phases were combined, dried over anhydrous sodium sulfate, filtered, and concentrated under reduced pressure. The residue was subjected to silica gel column chromatography (methanol/dichloromethane = 1%-10%) to give intermediate **1-2** (15.5 g, yield: 72.2%, brown solid). MS (ESI) m/z = 276.0 $[M+H]^+$.

1.2. Synthesis of intermediate **1-3**

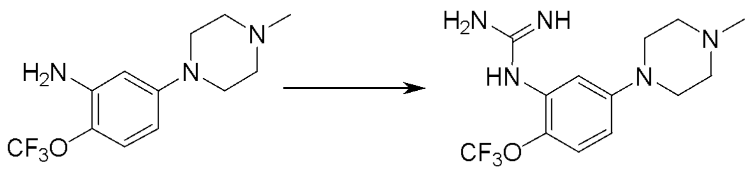

**1-2** **1-3**

Intermediate **1-2** (0.372 g, 1.35 mmol) was dissolved in 6 N HCl (2 mL), and cyanamide (0.455 g, 10.8 mmol) was added. The mixture was allowed to react at 80 °C for 12 h. The reaction solution was concentrated under reduced pressure, and the residue was subjected to column chromatography (C18, acetonitrile/water = 0%-10%) to give intermediate **1-3** (0.19 g, yield: 45%, brown solid). MS (ESI) m/z = 318.1 $[M+H]^+$.

1.3. Synthesis of intermediate **1-5**

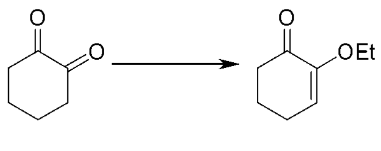

**1-4** **1-5**

Intermediate **1-4** (100 g, 892 mmol) was dissolved in toluene (1000 mL) and ethanol (123 g, 2680 mmol), and PTSA (15.4 g, 89.2 mmol) was added. The mixture was allowed to react at 120 °C for 12 h. Water (1000 mL × 2) was added for extraction. The organic phase was collected, dried over anhydrous sodium sulfate, filtered, and concentrated under reduced pressure. The residue was subjected to silica gel column chromatography (ethyl acetate/petroleum ether = 0%-50%) to give intermediate **1-5** (57.9 g, yield: 46.4%, oily substance).

1.4. Synthesis of intermediate **1-6**

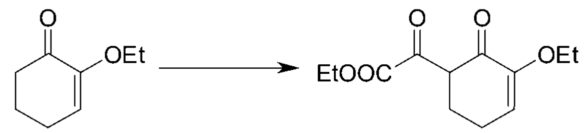

1-5 1-6

Intermediate **1-5** (30.0 g, 214 mmol) was dissolved in tetrahydrofuran (450 mL), and LiHMDS (1 M, 256 mL) was added dropwise at -50.0 °C. The mixture was stirred for 30 min. Diethyl oxalate (37.8 g, 258 mmol) was added, and the mixture was allowed to react at room temperature for 16 h. Saturated ammonium chloride (500 mL) was added to quench the reaction, and then ethyl acetate (50 mL × 3) was added for extraction. The organic phases were combined, dried over anhydrous sodium sulfate, filtered, and concentrated under reduced pressure. The residue was subjected to silica gel column chromatography (ethyl acetate/petroleum ether = 0%-20%) to give intermediate **1-6** (25.0 g, yield: 48.6%, oily substance). MS (ESI) m/z = 241.1 $[M+H]^+$.

1.5. Synthesis of intermediate **1-7**

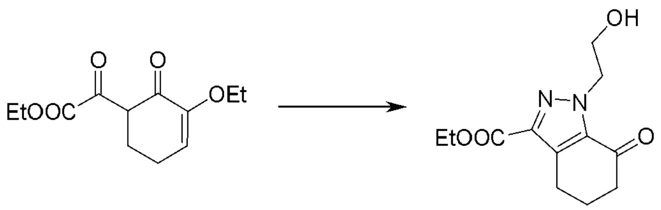

1-6 1-7

Intermediate **1-6** (25.0 g, 104 mmol) was dissolved in acetic acid (120 mL), and hydroxyethylhydrazine (8.16 g, 107 mmol) was added. The mixture was allowed to react at room temperature for 15 h. Water (500 mL) was added to quench the reaction, then saturated $Na_2CO_3$ (800 mL) was added, and dichloromethane (800 mL × 3) was added for extraction. The organic phases were combined, dried over anhydrous sodium sulfate, filtered, and concentrated under reduced pressure. The residue was subjected to silica gel column chromatography (ethyl acetate/petroleum ether = 0%-5%) to give intermediate **1-7** (19.9 g, yield: 76%, yellow solid). MS (ESI) m/z = 253.0 $[M+H]^+$.

1.6. Synthesis of intermediate **1-8**

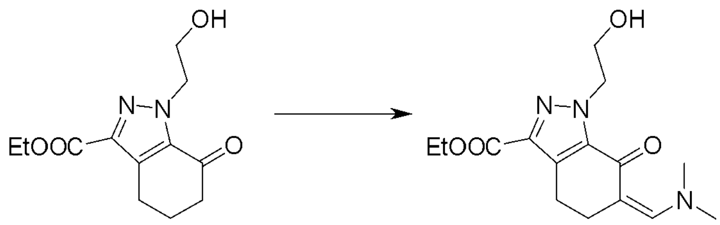

1-7 1-8

Intermediate **1-7** (10.0 g, 39.6 mmol) was dissolved in DMF-DMA (100 mL), and the resulting solution was allowed to react at 110 °C for 12 h. The reaction solution was concentrated under reduced pressure, and the residue was dissolved in methyl tert-butyl ether. The resulting solution was stirred for 3 h and then filtered. The filter cake was collected and dried to give intermediate **1-8** (6.0 g, crude product), which was used directly in the next step.

1.7. Synthesis of intermediate **1-9**

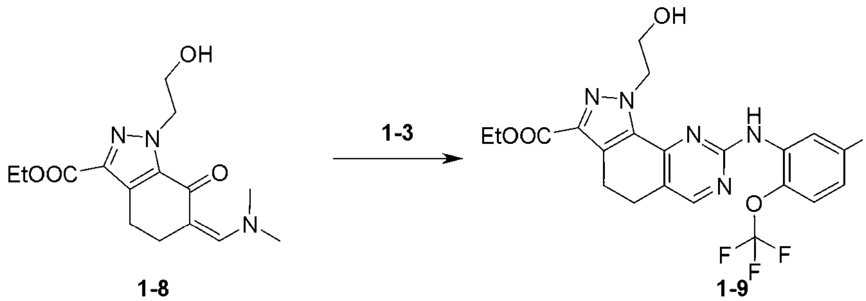


1-8 1-9

Intermediate **1-8** (1.95 g, 6.34 mmol) and intermediate **1-3** (2.03 g, 6.41 mmol) were dissolved in DMF (20 mL), and the resulting solution was allowed to react at 110 °C for 12 h. The reaction solution was concentrated under reduced pressure, and the residue was subjected to silica gel column chromatography (ethyl acetate/petroleum ether = 0%-5%) to give intermediate **1-9** (1.90 g, yield: 53.3%, yellow solid). MS (ESI) m/z = 562.2 $[M+H]^+$.

1.8. Synthesis of intermediate **1-10**

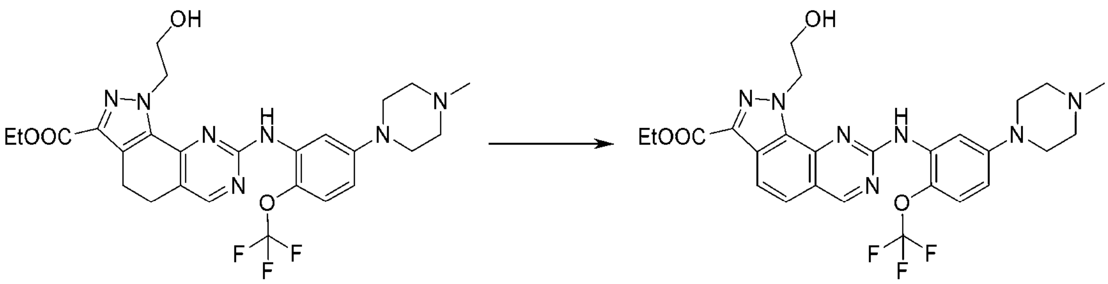

1-9 1-10

Intermediate **1-9** (100 mg, 178 μmol) and DDQ (100 mg, 440 μmol) were dissolved in dioxane (20 mL), and the resulting solution was allowed to react at 100 °C for 12 h. Saturated $NaHCO_3$ (20 mL) was added to quench the reaction, and then ethyl acetate (20 mL × 3) was added for extraction. The organic phases were combined, dried over anhydrous sodium sulfate, filtered, and concentrated under reduced pressure. The residue was purified by preparative liquid chromatography (C18 150 × 40 mm × 10 μm; mobile phase: [water (TFA)-acetonitrile]; B%: 25%-55%, 10 min) to give intermediate **1-10** (10 mg, yield: 10%, brown solid). MS (ESI) m/z = 560.1 $[M+H]^+$.

1.9. Synthesis of intermediate **1-11**

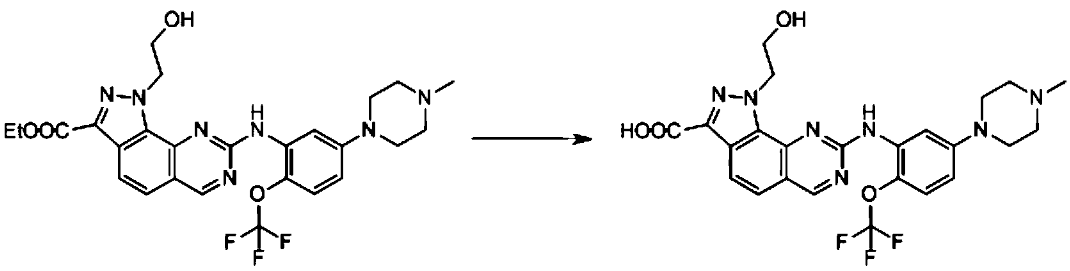

1-10 1-11

Intermediate **1-10** (74 g, 132.25 mmol) was taken, and acetonitrile (740 mL) was added, followed by the addition of a 1 mol/L aqueous hydrochloric acid solution (264 mL, prepared by dissolving 22 mL of hydrochloric acid in 242 mL of water). The mixture was heated to 60 °C, allowed to react for 1 h, then cooled to room temperature, stirred for 1.5 h, and filtered under vacuum. The filter cake was washed with an appropriate amount of 95% ethanol-water and then dried under reduced pressure in an oven at 55 °C to give a hydrochloride of **1-10** (37 g, yield: 50%, off-white solid). The hydrochloride of intermediate **1-10** (102 g, 171.14 mmol) was taken, and methanol (1.15 L) was added, followed by the addition of a 1 mol/L

aqueous lithium hydroxide solution (856 mL, prepared by dissolving 35.9 g of lithium hydroxide monohydrate in 856 mL of water). The mixture was heated to 60 °C, allowed to react for 3 h, and then cooled to room temperature. A 1 mol/L aqueous hydrochloric acid solution (740 mL, prepared by dissolving 61.7 mL of concentrated hydrochloric acid in 678.7 mL of water) was added, and the resulting mixture was stirred for 0.5 h, then concentrated under reduced pressure to remove the majority of the methanol solvent, and filtered under vacuum. The filter cake was washed with 100 mL of water and then dried under reduced pressure in an oven at 40 °C to give intermediate **1-11** (90 g, yield: 98.9%, white solid).

1.10. Synthesis and characterization of compound of formula I

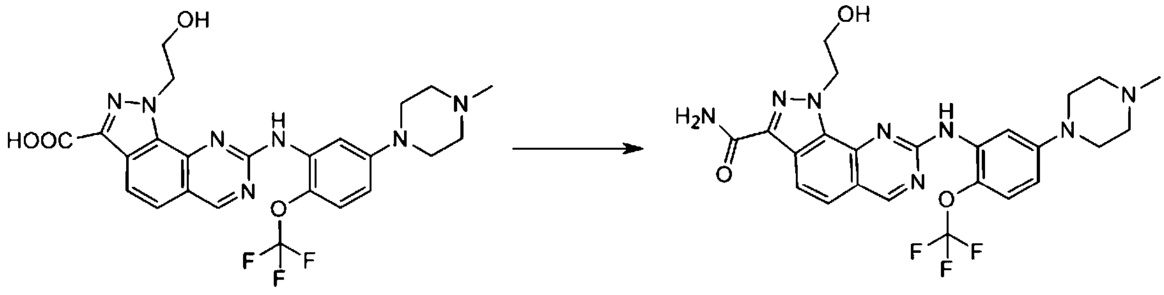

**1-11** Formula I

Intermediate **1-11** (80 g, 154.28 mmol) was taken, and ammonium chloride (24.76 g, 462.84 mmol) and N,N-diisopropylethylamine (59.8 g, 462.84 mmol) were added. Then, HATU (64.5 g, 169.7 mmol) was added under an ice bath, and the mixture was stirred at room temperature for 0.5 h, followed by the addition of an aqueous sodium hydroxide solution (3.28 L, prepared by dissolving 30.9 g of sodium hydroxide in 3.28 L of water). After the addition was completed, the resulting mixture was stirred at room temperature for 1.5 h and then filtered under vacuum. The filter cake was washed sequentially with 80 mL of water and 80 mL of methyl tert-butyl ether, and then dried under reduced pressure in an oven at 50 °C to give a crude product of the compound of formula I (76.6 g, yield: 93.6%, white solid). The crude product of the compound of formula I (12 g, 22.6 mmol) was taken, and 95% ethanol (120 mL) was added. The mixture was heated to 60 °C, stirred for 1 h, then cooled to room temperature, stirred for 3 h, and filtered under vacuum. The filter cake was washed with an appropriate amount of 95% ethanol-water and then dried under reduced pressure in an oven at 55 °C to give the compound of formula I (9.87 g, yield: 82.3%, white solid). MS (ESI) m/z = 531.2 [M+H]+. 1H NMR (400 MHz, DMSO-d6) δ 9.56 (s, 1H), 9.34 (s, 1H), 7.89 (d, J = 8.6 Hz, 1H), 7.67 (d, J = 8.8 Hz, 1H), 7.33 - 7.20 (m, 2H), 6.88 - 6.91 (m, 1H), 4.91 (t, J = 4.9 Hz, 2H), 4.65 (d, J = 5.3 Hz, 1H), 4.41 (q, J = 7.1 Hz, 2H), 3.70 - 3.54 (m, 3H), 3.28 - 3.10 (m, 3H), 3.10 - 3.09 (m, 1H), 3.16 - 3.09 (m, 1H), 2.34 - 2.19 (m, 3H), 1.39 (t, J = 7.1 Hz, 3H), 1.36 - 1.36 (m, 1H), 1.31 (t, J = 7.1 Hz, 1H), 1.26 - 1.12 (m, 1H).

The XRPD pattern of the compound of formula I (FIG. 1-1) shows that the compound was a crystal with relatively high crystallinity, designated as crystal form A. The characteristic peaks are shown in Table 1, and the purity was 98.33 area%. The TGA/DSC results (FIG. 1-2) show that the sample exhibited one sharp endothermic peak at 226.5 °C (onset temperature), and had a weight loss of 0.2% when heated from room temperature to 200 °C. The $^1$H NMR spectrum (FIG. 1-3, DMSO-d6) was consistent with the structure of the compound of formula I, and no significant solvent residue signals were observed. In view of the small weight loss in TGA and the presence of only one melting peak in DSC, it can be known that the crystal form A is an anhydrous crystal form.

Table 1. Analytical data of the XRPD pattern of the crystal form A of the compound of formula I

| No. | 2θ±0.2 (°) | Relative intensity (%) | No. | 2θ±0.2 (°) | Relative intensity (%) |
|---|---|---|---|---|---|
| 1 | 4.807 | 34.60 | 14 | 19.000 | 18.60 |
| 2 | 6.660 | 4.10 | 15 | 19.247 | 18.70 |
| 3 | 8.532 | 28.20 | 16 | 19.780 | 9.30 |
| 4 | 9.072 | 10.40 | 17 | 20.555 | 6.30 |
| 5 | 10.337 | 6.80 | 18 | 21.436 | 10.90 |
| 6 | 10.640 | 8.80 | 19 | 22.238 | 12.10 |
| 7 | 11.645 | 13.70 | 20 | 24.851 | 5.90 |

(continued)

| No. | 2θ±0.2 (°) | Relative intensity (%) | No. | 2θ±0.2 (°) | Relative intensity (%) |
|---|---|---|---|---|---|
| 8 | 13.272 | 10.90 | 21 | 25.514 | 23.60 |
| 9 | 13.973 | 7.10 | 22 | 25.866 | 15.90 |
| 10 | 15.160 | 100.00 | 23 | 26.765 | 5.00 |
| 11 | 16.768 | 15.10 | 24 | 29.128 | 2.00 |
| 12 | 17.520 | 14.80 | 25 | 30.559 | 4.40 |
| 13 | 18.347 | 18.30 | | | |

**Example 2. Crystal Form A of Maleate of Compound of Formula I**

91.82 mg of maleic acid and 399.55 mg of the compound of formula I (the feeding molar ratio of maleic acid to the compound of formula I was 1.05) were weighed into a 20 mL glass vial, and 5 mL of ethyl acetate was added. The mixture was suspended and stirred at room temperature for about 2 days, and then filtered under vacuum. The filter cake was rinsed with ethyl acetate and then dried under vacuum at 40 °C for 3 h to give the crystal form A of the maleate of the compound of formula I (464.11 mg, chemical purity: 98.38 area%). The XRPD pattern (FIG. 2-1) indicates relatively high crystallinity, and the characteristic peak positions are shown in Table 2. The TGA/DSC results (FIG. 2-2) show that the sample had a weight loss of 1.0% (corresponding to a weight loss of 1.4% for a hemihydrate) when heated from room temperature to 150 °C, and exhibited one endothermic signal at 214.4 °C (peak temperature). The $^1$H NMR results (FIG. 2-3) indicate that in the sample, the molar ratio of maleic acid to the compound of formula I was 1.0:1, and the molar ratio of the residual solvent ethyl acetate to the compound of formula I was 0.04 (about 0.5 wt%). Based on the above characterization results, it can be known that the crystal form A of the maleate is an anhydrous crystal form of the monomaleate.

Table 2. Analytical data of the XRPD pattern of the crystal form A of the maleate of the compound of formula I

| No. | 2θ±0.2 (°) | Relative intensity (%) | No. | 2θ±0.2 (°) | Relative intensity (%) |
|---|---|---|---|---|---|
| 1 | 6.883 | 22.30 | 21 | 21.790 | 14.50 |
| 2 | 7.431 | 10.30 | 22 | 22.330 | 6.60 |
| 3 | 8.455 | 78.90 | 23 | 23.052 | 16.40 |
| 4 | 8.798 | 8.60 | 24 | 23.818 | 20.10 |
| 5 | 10.366 | 11.70 | 25 | 24.127 | 18.70 |
| 6 | 12.313 | 32.70 | 26 | 24.787 | 39.20 |
| 7 | 12.802 | 59.20 | 27 | 25.243 | 5.60 |
| 8 | 13.239 | 8.40 | 28 | 25.493 | 18.00 |
| 9 | 13.894 | 64.50 | 29 | 25.823 | 24.00 |
| 10 | 14.640 | 22.80 | 30 | 26.760 | 14.10 |
| 11 | 14.908 | 14.40 | 31 | 27.185 | 4.10 |
| 12 | 15.691 | 5.60 | 32 | 27.571 | 6.40 |
| 13 | 17.051 | 46.90 | 33 | 28.060 | 6.50 |
| 14 | 17.369 | 26.60 | 34 | 28.303 | 20.00 |
| 15 | 17.713 | 44.20 | 35 | 29.653 | 8.10 |
| 16 | 18.025 | 7.60 | 36 | 29.824 | 16.70 |
| 17 | 19.029 | 47.40 | 37 | 30.373 | 8.10 |
| 18 | 19.592 | 17.70 | 38 | 31.810 | 3.60 |
| 19 | 20.917 | 98.50 | 39 | 32.690 | 3.50 |

(continued)

| No. | 20±0.2 (°) | Relative intensity (%) | No. | 2θ±0.2 (°) | Relative intensity (%) |
|---|---|---|---|---|---|
| 20 | 21.383 | 100.00 | 40 | 36.571 | 4.70 |

**Example 3. Crystal Form A of Fumarate of Compound of Formula I**

92.94 mg of fumaric acid and 404.63 mg of the compound of formula I (the feeding molar ratio of fumaric acid to the compound of formula I was 1.05) were weighed into a 20 mL glass vial, and 5 mL of 2-methyltetrahydrofuran was added. The mixture was suspended and stirred at room temperature for about 2 days, and then filtered under vacuum. The filter cake was rinsed with 2-methyltetrahydrofuran and then dried under vacuum at 40 °C for 3 h to give the crystal form A of the fumarate of the compound of formula I (448.02 mg, chemical purity: 98.36 area%). The XRPD pattern (FIG. 3-1) indicates high crystallinity, and the characteristic peaks are shown in Table 3. The TGA/DSC results (FIG. 3-2) show that the sample had a weight loss of 0.3% (corresponding to a weight loss of 1.4% for a hemihydrate) when heated from room temperature to 180 °C, and exhibited one endothermic signal at 266.6 °C (peak temperature). The $^1$H NMR results (FIG. 3-3) indicate that in the sample, the molar ratio of fumaric acid to the compound of formula I was 1.0:1, and the molar ratio of the residual solvent 2-methyltetrahydrofuran to the compound of formula I was 0.15 (about 2.0 wt%). Based on the above characterization results, it can be known that the crystal form A of the fumarate is an anhydrous crystal form of the monofumarate.

Table 3. Analytical data of the XRPD pattern of the crystal form A of the fumarate of the compound of formula I

| No. | 2θ±0.2 (°) | Relative intensity (%) | No. | 2θ±0.2 (°) | Relative intensity (%) |
|---|---|---|---|---|---|
| 1 | 6.813 | 13.50 | 17 | 20.768 | 27.10 |
| 2 | 7.931 | 46.40 | 18 | 21.636 | 89.30 |
| 3 | 11.204 | 13.40 | 19 | 22.285 | 40.20 |
| 4 | 11.937 | 3.80 | 20 | 22.641 | 69.40 |
| 5 | 12.635 | 34.70 | 21 | 22.902 | 75.60 |
| 6 | 12.970 | 16.40 | 22 | 23.597 | 42.60 |
| 7 | 13.761 | 34.50 | 23 | 23.963 | 19.00 |
| 8 | 14.265 | 4.50 | 24 | 24.234 | 50.30 |
| 9 | 15.752 | 100.00 | 25 | 25.033 | 17.50 |
| 10 | 16.012 | 53.20 | 26 | 25.576 | 20.80 |
| 11 | 16.972 | 34.60 | 27 | 27.332 | 7.60 |
| 12 | 17.545 | 10.50 | 28 | 27.948 | 49.30 |
| 13 | 17.983 | 26.40 | 29 | 29.020 | 29.30 |
| 14 | 19.481 | 36.20 | 30 | 29.329 | 40.70 |
| 15 | 19.719 | 31.00 | 31 | 35.562 | 4.40 |
| 16 | 20.426 | 71.40 | | | |

**Example 4. Crystal Form A of Succinate of Compound of Formula I**

406.28 mg of the compound of formula I and 94.92 mg of succinic acid (the feeding molar ratio of succinic acid to the compound of formula I was 1.05) were weighed into a 20 mL glass vial, and 5 mL of 2-MeTHF was added. The mixture was suspended and stirred at room temperature for about 2 days, and then filtered under vacuum. The filter cake was rinsed with 2-methyltetrahydrofuran and then dried under vacuum at 40 °C for 3 h to give the crystal form A of the succinate of the compound of formula I (441.58 mg, chemical purity: 98.39 area%). The XRPD pattern (FIG. 4-1) indicates relatively high crystallinity, and the characteristic peaks are shown in Table 4. The TGA/DSC results (FIG. 4-2) show that the sample had a weight loss of 0.4% (corresponding to a weight loss of 1.4% for a hemihydrate) when heated from room temperature to 150 °C, and exhibited one endothermic signal at 223.3 °C (peak temperature). The $^1$H NMR results (FIG. 4-3) show that in the sample, the molar ratio of succinic acid to the compound of formula I was 1.0:1, and the molar ratio of the residual solvent 2-methyltetrahydrofuran to the compound of formula I was 0.08 (about 1.1 wt%). Based on the above characterization results, it can be known that the crystal form A of the succinate is an anhydrous crystal form of the monosuccinate.

Table 4. Analytical data of the XRPD pattern of the crystal form A of the succinate of the compound of formula I

| No. | 2θ±0.2 (°) | Relative intensity (%) | No. | 2θ±0.2 (°) | Relative intensity (%) |
|---|---|---|---|---|---|
| 1 | 6.655 | 29.70 | 15 | 20.105 | 55.90 |
| 2 | 7.771 | 51.30 | 16 | 20.452 | 19.60 |
| 3 | 11.073 | 9.60 | 17 | 21.347 | 78.90 |
| 4 | 12.486 | 22.50 | 18 | 21.933 | 24.70 |
| 5 | 12.757 | 16.40 | 19 | 22.552 | 100.00 |
| 6 | 13.539 | 39.50 | 20 | 23.251 | 24.70 |
| 7 | 14.099 | 6.00 | 21 | 23.887 | 38.80 |
| 8 | 15.519 | 98.60 | 22 | 24.834 | 10.00 |
| 9 | 15.781 | 33.80 | 23 | 25.266 | 15.80 |
| 10 | 16.835 | 16.20 | 24 | 26.122 | 6.50 |
| 11 | 17.260 | 15.90 | 25 | 27.583 | 25.20 |
| 12 | 17.709 | 30.70 | 26 | 28.647 | 8.50 |
| 13 | 19.077 | 22.30 | 27 | 28.930 | 26.20 |
| 14 | 19.391 | 27.20 | | | |

**Example 5. Crystal Form B of Hemifumarate of Compound of Formula I**

An appropriate amount of the crystal form A of the monofumarate was weighed and completely dissolved in N-methylpyrrolidone (NMP). The resulting solution was filtered through a filter membrane (nylon membrane, with a pore size of 0.22 μm) to give a stock solution. The stock solution was aliquoted into 20 mL glass vials (each glass vial containing 20 mg of the sample). Under magnetic stirring conditions, methyl tert-butyl ether was gradually added to each glass vial until a solid appeared or the total volume of the solvent reached 9 mL. The mixture was then filtered under vacuum, and the filter cake was air-dried at room temperature to give the crystal form B of the hemifumarate of the compound of formula I.

The XRPD pattern (FIG. 5-1) indicates relatively high crystallinity, and the crystal form of the sample remained unchanged after drying at room temperature. The characteristic peaks are shown in Table 5. The TGA/DSC results (FIG. 5-2) show that the sample had a weight loss of 23.3% when heated from room temperature to 140 °C, and had a weight loss of 8.0% when further heated to 240 °C; the sample exhibited four endothermic signals at 92.5 °C, 105.1 °C, 170.5 °C, and 263.5 °C (peak temperatures). The $^1$H NMR spectrum (FIG. 5-3) shows that in the sample, the molar ratio of fumaric acid to the compound of formula I was 0.6:1, the molar ratio of N-methylpyrrolidone to the compound of formula I was 2.0:1 (about 24.7 wt%), and the molar ratio of methyl tert-butyl ether to the compound of formula I was 0.03 (about 0.3 wt%). The crystal form B of the hemifumarate was subjected to XRPD analysis after heating. After being heated to 140 °C, the sample transformed into the crystal form G of the hemifumarate (its characterization details are provided in Example 10); after being further heated to 200 °C, it transformed into the crystal form A of the monofumarate. Based on the characterization results, it can be known that the crystal form B of the hemifumarate is an NMP solvate of the hemifumarate.

Repeated preparation of crystal form B of hemifumarate: An appropriate amount of the crystal form A of the monofumarate was weighed and completely dissolved in NMP. The resulting solution was filtered through a filter membrane (nylon membrane, with a pore size of 0.22 μm) to give a stock solution. The stock solution was aliquoted into 4 mL glass vials (each glass vial containing 20 mg of the sample), which were then respectively placed into 20 mL glass vials each containing 4 mL of the anti-solvent acetone or isopropyl acetate. Each of the 20 mL glass vials was tightly capped and placed at room temperature until a solid was precipitated. The mixture was then filtered under vacuum, and the filter cake was dried under vacuum at room temperature to give the crystal form B of the hemifumarate. In the crystal form B of the hemifumarate obtained with acetone as the anti-solvent, the molar ratio of fumaric acid to the compound of formula I was 0.7:1, and the molar ratio of NMP to the compound of formula I was 0.9:1. In the crystal form B of the hemifumarate obtained with isopropyl acetate as the anti-solvent, the molar ratio of fumaric acid to the compound of formula I was 0.7:1, and the molar ratio of NMP to the compound of formula I was 1.1:1.

Table 5. Analytical data of the XRPD pattern of the crystal form B of the hemifumarate of the compound of formula I

| No. | 2θ±0.2 (°) | Relative intensity (%) | No. | 2θ±0.2 (°) | Relative intensity (%) |
|---|---|---|---|---|---|
| 1 | 6.260 | 2.90 | 11 | 19.796 | 4.60 |
| 2 | 6.927 | 1.70 | 12 | 21.119 | 2.20 |
| 3 | 8.570 | 6.40 | 13 | 23.390 | 2.00 |
| 4 | 12.256 | 3.80 | 14 | 24.838 | 100.00 |
| 5 | 12.545 | 6.20 | 15 | 26.132 | 14.20 |
| 6 | 13.561 | 1.00 | 16 | 26.736 | 31.90 |
| 7 | 15.084 | 12.80 | 17 | 27.529 | 4.60 |
| 8 | 15.501 | 10.30 | 18 | 33.127 | 1.10 |
| 9 | 17.311 | 9.30 | 19 | 33.944 | 2.10 |
| 10 | 17.965 | 3.00 | | | |

**Example 6. Crystal Form C of Hemifumarate of Compound of Formula I**

An appropriate amount of the crystal form A of the monofumarate was weighed and completely dissolved in NMP. The resulting solution was filtered through a filter membrane (nylon membrane, with a pore size of 0.22 μm) to give a stock solution. The stock solution was aliquoted into 20 mL glass vials (each glass vial containing about 20 mg of the sample). Under magnetic stirring conditions, water was gradually added to each glass vial until a solid appeared or the total volume of the solvent reached 9 mL. The mixture was then filtered under vacuum, and the filter cake was air-dried at room temperature to give the crystal form C of the hemifumarate of the compound of formula I.

The XRPD pattern (FIG. 6-1) indicates relatively high crystallinity, and the crystal form remained unchanged after drying at room temperature. The characteristic peaks are shown in Table 6. The TGA/DSC results (FIG. 6-2) show that the sample had a weight loss of 5.8% (corresponding to a weight loss of 2.9% for one mole of water) when heated from room temperature to 80 °C, and had a weight loss of 1.3% when further heated to 140 °C; the sample exhibited four endothermic signals at 68.7 °C, 115.6 °C, 226.0 °C, and 262.3 °C (peak temperatures), and one exothermic signal at 177.1 °C (peak temperature). The $^1$H NMR spectrum (FIG. 6-3) shows that in the sample, the molar ratio of fumaric acid to the compound of formula I was 0.5:1, and no residual NMP was observed. Based on the characterization results, it can be known that the crystal form C of the hemifumarate is a hydrate crystal form of the hemifumarate.

Repeated preparation of crystal form C of hemifumarate: An appropriate amount of the crystal form A of the monofumarate was weighed and completely dissolved in tetrahydrofuran/water (1:1). The resulting solution was filtered through a filter membrane (nylon membrane, with a pore size of 0.22 μm) to give a stock solution. The stock solution was aliquoted into 20 mL glass vials (each glass vial containing 20 mg of the sample). Under magnetic stirring conditions, the anti-solvent $H_2O$ was gradually added to each glass vial until a solid appeared or the total volume of the solvent reached 9 mL. The mixture was then filtered under vacuum, and the filter cake was dried under vacuum at room temperature to give the crystal form C of the hemifumarate. XRPD analysis was performed using Rigaku Smart SE. After being heated to 90 °C and 140 °C, the crystal form C of the hemifumarate transformed into the crystal form D of the hemifumarate; after being further heated to 200 °C, it transformed into the crystal form A of the fumarate and the crystal form A of the compound of formula I.

Table 6. Analytical data of the XRPD pattern of the crystal form C of the hemifumarate of the compound of formula I

| No. | 2θ±0.2 (°) | Relative intensity (%) | No. | 2θ±0.2 (°) | Relative intensity (%) |
|---|---|---|---|---|---|
| 1 | 6.637 | 26.70 | 17 | 20.121 | 19.00 |
| 2 | 7.553 | 100.00 | 18 | 20.609 | 22.00 |
| 3 | 9.346 | 5.20 | 19 | 20.966 | 16.20 |
| 4 | 11.403 | 4.60 | 20 | 21.533 | 10.80 |
| 5 | 12.329 | 7.40 | 21 | 22.303 | 7.70 |
| 6 | 12.716 | 61.10 | 22 | 22.755 | 29.40 |
| 7 | 13.066 | 4.90 | 23 | 23.415 | 16.80 |

(continued)

| No. | 2θ±0.2 (°) | Relative intensity (%) | No. | 2θ±0.2 (°) | Relative intensity (%) |
|---|---|---|---|---|---|
| 8 | 13.547 | 51.60 | 24 | 23.756 | 9.50 |
| 9 | 14.492 | 8.30 | 25 | 24.061 | 10.50 |
| 10 | 15.313 | 51.40 | 26 | 26.380 | 6.00 |
| 11 | 15.628 | 6.20 | 27 | 26.830 | 6.20 |
| 12 | 16.305 | 14.20 | 28 | 27.775 | 5.40 |
| 13 | 17.548 | 6.30 | 29 | 29.047 | 6.40 |
| 14 | 17.880 | 6.30 | 30 | 31.020 | 4.10 |
| 15 | 19.089 | 42.90 | 31 | 32.695 | 6.30 |
| 16 | 19.576 | 17.80 | | | |

**Example 7. Crystal Form D of Hemifumarate of Compound of Formula I**

A sample of the crystal form C of the hemifumarate was heated to 140 °C to give the crystal form D of the hemifumarate. XRPD analysis was performed using Rigaku Smart SE. The XRPD pattern of the crystal form D is shown in FIG. 7-1, and its characteristic peaks are shown in Table 7. The $^1$H NMR spectrum (FIG. 7-2) shows that in the sample, the molar ratio of fumaric acid to the compound of formula I was 0.5:1, and no other solvent residues were detected. The TGA/DSC results (FIG. 7-3) show that after being heated from room temperature to 150 °C, the sample had a weight loss of 0.8% (corresponding to a weight loss of 1.5% for half a mole of water); the sample exhibited three endothermic signals at 62.5 °C, 227.6 °C, and 263.6 °C (peak temperatures), and one exothermic signal at 170.8 °C (peak temperature). After the crystal form D of the hemifumarate was heated to 100 °C, its crystal form remained unchanged. Based on the above characterization results, it can be known that the crystal form D of the hemifumarate is an anhydrous crystal form or a channel hydrate of the hemifumarate.

Table 7. Analytical data of the XRPD pattern of the crystal form D of the hemifumarate of the compound of formula I

| No. | 2θ±0.2 (°) | Relative intensity (%) | No. | 2θ±0.2 (°) | Relative intensity (%) |
|---|---|---|---|---|---|
| 1 | 6.881 | 100.00 | 5 | 13.831 | 50.80 |
| 2 | 8.103 | 31.30 | 6 | 19.569 | 24.30 |
| 3 | 12.572 | 31.20 | 7 | 20.533 | 24.70 |
| 4 | 13.357 | 13.30 | 8 | 24.021 | 28.90 |

**Example 8. Crystal Form E of Hemifumarate of Compound of Formula I**

An appropriate amount of the crystal form A of the monofumarate was weighed and completely dissolved in N,N-dimethylacetamide (DMAc). The resulting solution was filtered through a filter membrane (nylon membrane, with a pore size of 0.22 μm) to give a stock solution. The stock solution was aliquoted into 4 mL glass vials (each glass vial containing 20 mg of the sample), which were then respectively placed into 20 mL glass vials each containing 4 mL of $CHCl_3$. Each of the 20 mL glass vials was tightly capped and placed at room temperature until a solid was precipitated.

The resulting solid was subjected to XRPD characterization, and the results are shown in FIG. 8-1. The crystal form of the sample remained unchanged after drying at room temperature. The characteristic peaks are shown in Table 8. The TGA/DSC results (FIG. 8-2) show that the sample had a weight loss of 3.5% when heated from room temperature to 100 °C, and had a weight loss of 11.5% when further heated to 200 °C; the sample exhibited six endothermic signals at 62.6 °C, 154.9 °C, 185.7 °C, 226.6 °C, 235.5 °C, and 264.5 °C (peak temperatures). The $^1$H NMR spectrum (FIG. 8-3) shows that in the sample, the molar ratio of fumaric acid to the compound of formula I was 0.7:1, the molar ratio of DMAc to the compound of formula I was 0.4:1 (about 5.1 wt%), and the molar ratio of $CHCl_3$ to the compound of formula I was 0.3:1 (about 5.2 wt%). After being heated to 120 °C, the crystal form E of the hemifumarate exhibited multiple peaks; after being heated to 200 °C, it transformed into the crystal form A of the monofumarate with multiple peaks (the multiple peaks are all marked with asterisks); after being further heated to 240 °C, it transformed into the crystal form A of the monofumarate (FIG. 8-4). In view of the above characterization results, it can be known that the crystal form E of the hemifumarate is a DMAc/$CHCl_3$ co-solvate of the hemifumarate.

Table 8. Analytical data of the XRPD pattern of the crystal form E of the hemifumarate of the compound of formula I

| No. | 2θ±0.2 (°) | Relative intensity (%) | No. | 2θ±0.2 (°) | Relative intensity (%) |
|---|---|---|---|---|---|
| 1 | 6.188 | 7.90 | 17 | 23.726 | 0.80 |
| 2 | 8.708 | 1.90 | 18 | 23.970 | 0.70 |
| 3 | 11.249 | 0.80 | 19 | 25.017 | 25.40 |
| 4 | 12.226 | 1.40 | 20 | 25.275 | 10.40 |
| 5 | 12.492 | 100.00 | 21 | 26.603 | 16.60 |
| 6 | 13.568 | 0.70 | 22 | 26.948 | 0.80 |
| 7 | 15.054 | 0.70 | 23 | 27.465 | 0.40 |
| 8 | 15.473 | 5.70 | 24 | 28.099 | 0.40 |
| 9 | 15.965 | 0.50 | 25 | 28.569 | 0.40 |
| 10 | 17.729 | 10.20 | 26 | 30.394 | 0.30 |
| 11 | 18.851 | 0.70 | 27 | 31.795 | 4.80 |
| 12 | 19.596 | 1.20 | 28 | 32.271 | 0.40 |
| 13 | 20.002 | 1.70 | 29 | 33.456 | 0.40 |
| 14 | 20.773 | 1.20 | 30 | 33.974 | 0.50 |
| 15 | 21.645 | 0.80 | 31 | 35.720 | 0.40 |
| 16 | 22.726 | 0.70 | 32 | 38.395 | 3.40 |

**Example 9. Crystal Form F of Hemifumarate of Compound of Formula I**

An appropriate amount of the crystal form A of the monofumarate was weighed and completely dissolved in DMAc. The resulting solution was filtered through a filter membrane (nylon membrane, with a pore size of 0.22 μm) to give a stock solution. The stock solution was aliquoted into 4 mL glass vials (each glass vial containing 20 mg of the sample), which were then respectively placed into 20 mL glass vials each containing 4 mL of the anti-solvent methyl tert-butyl ether. Each of the 20 mL glass vials was tightly capped and placed at room temperature until a solid was precipitated.

The resulting solid was subjected to XRPD characterization, and the results are shown in FIG. 9-1. The crystal form of the sample remained unchanged after drying at room temperature. The characteristic peaks are shown in Table 9. The TGA/DSC results (FIG. 9-2) show that the sample had a weight loss of 4.2% when heated from room temperature to 100 °C, and had a weight loss of 7.7% when further heated to 200 °C; the sample exhibited four endothermic signals at 58.8 °C, 199.0 °C, 227.3 °C, and 263.2 °C (peak temperatures). The $^1$H NMR spectrum (FIG. 9-3) shows that in the sample, the molar ratio of fumaric acid to the compound of formula I was 0.5:1, and the molar ratio of DMAc to the compound of formula I was 0.5:1 (about 6.9 wt%). After being heated to 120 °C, the crystal form F of the hemifumarate remained unchanged; after being heated to 210 °C, it transformed into the crystal form A of the monofumarate and the crystal form A of the compound of formula I; after being further heated to 240 °C, it transformed into the crystal form A of the monofumarate. The sample heated to 120 °C was subjected to $^1$H NMR analysis. The molar ratio of DMAc to the compound of formula I in the sample remained 0.5. Since the crystal form F of the hemifumarate underwent crystal transformation after being heated to 210 °C, and DMAc was still observed after heating to 120 °C, it can be known that the resulting crystal form F of the hemifumarate was a DMAc solvate of the hemifumarate.

Repeated preparation of DMAc solvate of crystal form F of hemifumarate: An appropriate amount of the crystal form A of the monofumarate was weighed and completely dissolved in DMAc. The resulting solution was filtered through a filter membrane (nylon membrane, with a pore size of 0.22 μm) to give a stock solution. The stock solution was aliquoted into 4 mL glass vials (each glass vial containing 20 mg of the sample), which were then respectively placed into 20 mL glass vials each containing 4 mL of the anti-solvent ethanol. Each of the 20 mL glass vials was tightly capped and placed at room temperature, resulting in the precipitation of a solid, which was the DMAc solvate of the crystal form F of the hemifumarate. Analysis showed that the molar ratio of fumaric acid to the compound of formula I was 0.6:1.

Table 9. Analytical data of the XRPD pattern of the crystal form F of the hemifumarate of the compound of formula I

| No. | 2θ±0.2 (°) | Relative intensity (%) | No. | 2θ±0.2 (°) | Relative intensity (%) |
|---|---|---|---|---|---|
| 1 | 6.634 | 1.80 | 15 | 20.347 | 1.30 |
| 2 | 8.185 | 7.10 | 16 | 20.898 | 2.40 |
| 3 | 8.866 | 15.50 | 17 | 21.589 | 3.20 |
| 4 | 11.990 | 2.00 | 18 | 22.679 | 0.60 |
| 5 | 12.423 | 1.50 | 19 | 23.550 | 1.00 |
| 6 | 12.914 | 0.90 | 20 | 24.784 | 3.70 |
| 7 | 13.449 | 5.70 | 21 | 25.199 | 100.00 |
| 8 | 14.456 | 1.90 | 22 | 26.157 | 56.50 |
| 9 | 15.810 | 4.80 | 23 | 27.140 | 1.00 |
| 10 | 16.651 | 25.00 | 24 | 28.782 | 3.10 |
| 11 | 16.841 | 5.80 | 25 | 34.095 | 3.70 |
| 12 | 18.058 | 1.80 | 26 | 35.497 | 2.00 |
| 13 | 18.910 | 4.10 | 27 | 38.478 | 1.00 |
| 14 | 19.618 | 1.70 | | | |

**Example 10. Crystal Form G of Hemifumarate of Compound of Formula** I

The crystal form B of the hemifumarate prepared in Example 5 was heated to 140 °C to give the crystal form G of the hemifumarate. The XRPD pattern of the sample is shown in FIG. 10-1, and its characteristic peaks are shown in Table 10. The TGA/DSC results (FIG. 10-2) show that the sample had a weight loss of 6.4% when heated from room temperature to 220 °C, and exhibited three endothermic signals at 196.5 °C, 225.9 °C, and 263.3 °C (peak temperatures). The $^{1}H$ NMR spectrum (FIG. 10-3) shows that in the sample, the molar ratio of fumaric acid to the compound of formula I was 0.6:1, and the molar ratio of NMP to the compound of formula I was 0.5:1 (about 6.2 wt%). In view of the weight loss in TGA, the presence of solvent in NMR, the transformation of the crystal form B of the hemifumarate into the crystal form G of the hemifumarate after heating to 140 °C, and its transformation into the crystal form A of the monofumarate after heating to 200 °C (see Example 5), it can be known that the crystal form G of the hemifumarate is an NMP solvate of the hemifumarate.

Table 10. Analytical data of the XRPD pattern of the crystal form G of the hemifumarate of the compound of formula I

| No. | 2θ±0.2 (°) | Relative intensity (%) | No. | 2θ±0.2 (°) | Relative intensity (%) |
|---|---|---|---|---|---|
| 1 | 6.707 | 28.80 | 19 | 18.923 | 36.70 |
| 2 | 7.294 | 31.30 | 20 | 19.440 | 16.20 |
| 3 | 7.954 | 14.80 | 21 | 19.720 | 15.20 |
| 4 | 8.293 | 10.10 | 22 | 20.442 | 34.10 |
| 5 | 8.925 | 41.60 | 23 | 21.133 | 73.90 |
| 6 | 9.845 | 7.10 | 24 | 21.632 | 33.50 |
| 7 | 12.025 | 58.20 | 25 | 22.261 | 17.60 |
| 8 | 12.948 | 93.30 | 26 | 22.637 | 17.60 |
| 9 | 13.525 | 75.40 | 27 | 22.914 | 26.50 |
| 10 | 13.792 | 19.80 | 28 | 23.547 | 82.00 |
| 11 | 14.437 | 15.90 | 29 | 24.270 | 17.80 |
| 12 | 15.510 | 38.20 | 30 | 24.544 | 35.40 |
| 13 | 15.763 | 100.00 | 31 | 25.164 | 86.30 |

(continued)

| No. | 2θ±0.2 (°) | Relative intensity (%) | No. | 2θ±0.2 (°) | Relative intensity (%) |
|---|---|---|---|---|---|
| 14 | 16.016 | 30.20 | 32 | 26.101 | 90.50 |
| 15 | 16.417 | 38.40 | 33 | 27.908 | 16.60 |
| 16 | 16.793 | 70.30 | 34 | 28.747 | 14.90 |
| 17 | 17.497 | 11.40 | 35 | 29.349 | 12.30 |
| 18 | 18.129 | 15.90 | | | |

**Test Example 1. Inhibitory Activity Against PLK1 Kinase**

1. Test materials: PLK1 kinase (Carnabio #05-157)

Detection solution: PerkinElmer #TRF0218-M
Detection instrument: PerkinElmer EnVision

2. Test method:

a. The test compound was serially diluted in a 1:3 ratio on a 384-well plate using an Echo instrument to obtain a total of 10 concentration points, with the highest concentration being 1 μM. Each concentration was tested in duplicate.
b. A mixture of PLK1 kinase and peptide substrate was added to the assay plate (containing control samples) at 5 μL/well. For each assay plate, GSK461364 was set as a positive control, and a DMSO solvent group was set as a negative control.
c. The assay plate was centrifuged at 1000 rpm for about 15 s and then incubated at 23 °C for 15 min.
d. An ATP solution was added at 5 μL/well to initiate the reaction.
e. The assay plate was centrifuged at 1000 rpm for about 15 s, then sealed with a thin film, and incubated at 23 °C for a period of time.
f. The detection solution was then added to all wells of the assay plate.
g. The assay plate was centrifuged at 1000 rpm for about 15 s, then sealed with a thin film, and incubated at 23 °C for 60 min.
h. The assay plate was read on EnVision. The test results were analyzed using XLFIT5 software.

3. Test results:

The $IC_{50}$ and $I_{max}$ (%) values for the inhibitory activity of the compound of the present disclosure against PLK1 kinase are shown in Table 11. $I_{max}$ represents the maximum effect induced by the compound at the test concentration (S/B 8.52, Z factor 0.94).

Table 11. PLK1 kinase inhibition test results

| Compound No. | $IC_{50}$ (nM) | $I_{max}$ (%) |
|---|---|---|
| Formula I | 1.81 | 99.35 |

Conclusion: The compound of formula I exhibited a significant inhibitory effect on PLK1 kinase.

**Test Example 2. Inhibitory Activity Against Proliferation of HCT116 Cells**

1. Test materials:

Cell line: HCT116 cells
Cell culture medium: RPMI1640 + 10% FBS (GBICO)
Cell culture plate: 96-well plate (Shanghai Jing An Biological Technology Co., Ltd.)
Detection kit: ATPlite 1step Luminescence (PerkinElmer)
Detection instrument: BioTek multimode microplate reader

2. Test method:
HCT116 cells in the logarithmic growth phase were seeded into a white-walled, clear-bottom 96-well plate at a density of 720 cells/well and cultured overnight in an incubator at 37 °C with 5% $CO_2$. The next day, the test compound at a concentration of 10 μM was serially diluted 3-fold to obtain 9 concentration points, and then the dilutions were added to the cells. Duplicate wells were set up for each concentration. Blank culture medium was used as a negative control, and onvansertib at the same concentration was used as a positive control. After the drug addition, the cells were cultured in the incubator at 37 °C with 5% $CO_2$ for another 72 h, and then the ATPlite 1step Luminescence reagent was added to the cells in a volume equal to that of the cell solution. The mixture was incubated in the dark at room temperature for 3 min and shaken on a micro-oscillator at 500 rpm for 2 min. The luminescence intensity was measured using a microplate reader, and then the cell inhibition rate was calculated.

$$\text{Cell inhibition rate (\%)} = [100 - (\text{Lum}_{\text{test sample}} - \text{Lum}_{\text{culture medium}})/(\text{Lum}_{\text{negative control}} - \text{Lum}_{\text{culture medium}}) \times 100]\%.$$

The data were processed using GraphPad Prism 7.0 to obtain the cell inhibition rate curve and calculate the $IC_{50}$.
3. Test results:
The half-maximal inhibitory concentration $IC_{50}$ of the compound of formula I against the proliferation of HCT116 cells was 0.051 μM, indicating that the compound can effectively inhibit the proliferation of HCT116 cells.

**Test Example 3. Evaluation of Permeability of Test Substance and Transporter Substrate**

1. Test materials:
Caco-2 cells were seeded into a Transwell-96 well plate at a density of $1 \times 10^5$ cells/cm$^2$. The cells were cultured in a carbon dioxide incubator for 21-28 days before being used in the transport experiment. During the culture period, the culture medium was changed every four to five days.
2. Test method:
A Hank's balanced salt buffer containing 10 mM HEPES (pH 7.40±0.05) was used as a transport buffer. The culture medium in the plate was removed, and the cells were rinsed twice with pre-warmed transport buffer (using approximately 75 μL per apical chamber and 40 mL per basolateral receiver plate for each rinse). The dosing solution and the receiving solution were separately added to the corresponding well positions of the cell plate (75 μL per apical chamber and 250 μL per basolateral chamber) to initiate the bidirectional transport experiment (apical-to-basolateral (A-B) and basolateral-to-apical (B-A)).

The test concentration of the test sample was 5.00 μM. Duplicate wells were set up for each administered concentration. The test concentration of digoxin was 10.0 μM (administered bidirectionally). The test concentrations of nadolol and metoprolol were both 2.00 μM (administered unidirectionally (A-B direction)). Duplicate wells were also set up for each of the three control compounds. After the sample addition, the cell plate was incubated at 37±1 °C with 5% $CO_2$ and saturated humidity for 120 min.
The initial dosing solution served as the $T_0$ sample; after the sample addition, it was taken and mixed with the transport buffer and a stop solution in a certain ratio. After incubation for 120 min, the final samples were collected from the donor and receiver ends, and were similarly mixed with the transport buffer and the stop solution in a certain ratio.
All samples were vortexed and then centrifuged at 3220× g at 20 °C for 20 min. An appropriate volume of the supernatant was transferred to a sample analysis plate. After plate sealing, if the samples were not analyzed immediately, they were stored at 2-8 °C. Analysis was performed using the LC-MS/MS method.
The integrity of the Caco-2 cell layers was tested using the lucifer yellow rejection assay.

(1) Data analysis:

The apparent permeability coefficient ($P_{app}$, cm/s), the efflux ratio (ER), and the solution recovery (%) (%Solution Recovery) were calculated using the following formulas.

$$P_{app} = \frac{V_R}{Area \times Time} \times \frac{[drug]_{recevier}}{[drug]_{initial, donor}} = \frac{V_R}{Area \times Time} \times \frac{C_R}{C_0}$$

$$ER = \frac{P_{app}(B-A)}{P_{app}(A-B)}$$

$$\%SolutionRecovery = \frac{C_R \times V_R + C_D \times V_D}{C_0 \times V_D} \times 100$$

$V_R$ represents the volume of the solution at the receiver end (0.075 mL for side A and 0.25 mL for side B); Area represents the relative surface area of the cell monolayer (0.0804 $cm^2$); Time represents the incubation time (7200 s); $C_0$ represents the peak area ratio of the initial concentration (nM) of the test sample or the control compound at the donor end; $V_D$ represents the volume at the donor end (0.075 mL for side A and 0.25 mL for side B); $C_D$ and $C_R$ represent the peak area ratios of the final concentrations (nM) of the test sample or the control compound at the donor end and the receiver end, respectively;
The permeation rate of Lucifer Yellow (%Lucifer Yellow) was calculated using the following formula:

$$\%\ Lucifer\ \ Yellow = \frac{V_{Basolatera\ \ l} \times RFU_{Basolatera\ \ l}}{V_{Apical} \times RFU_{Apical} + V_{Basolatera\ \ l} \times RFU_{Basolatera\ \ l}} \times 100$$

$RFU_{Apical}$ and $RFU_{Basolateral}$ represent the relative fluorescence intensities of Lucifer Yellow at the apical and basolateral sides, respectively. $V_{Apical}$ and $V_{Basolateral}$ represent the sample loading volumes at the apical and basolateral sides, respectively (0.075 mL and 0.25 mL, respectively).

(2) Classification criteria:

| Parameter | Classification | Criteria |
|---|---|---|
| Permeability | Low | $P_{app}$ (A -B) ≤ 0.500 (× $10^{-6}$ cm/s) |
| | Medium | 0.500 < $P_{app}$ (A-B) < 2.50 (× $10^{-6}$ cm/s) |
| | High | $P_{app}$ (A-B) ≥ 2.50 (× $10^{-6}$ cm/s) |
| Efflux transporter substrate | Likely | $ER_a$ ≥ 2.00 |
| | Unlikely/Not | $ER_a$ < 2.00 |

3. Test results: The permeability results of the compound of formula I in Caco-2 cells are shown in Table 12.

Table 12. Permeability results in Caco-2 cells

| Compound No. | Mean $P_{app}$ ($10^{-6}$ cm/s) | | Efflux ratio | Classification | |
|---|---|---|---|---|---|
| | A-B | B-A | | Permeability | Efflux transporter substrate |
| Formula I | 0.965 | 20.8 | 21.5 | Medium | Likely |

**Test Example 4. Evaluation of Metabolic Stability in Liver Microsomes**

1. Reagents:

| Name | Content | Batch No. | Source |
|---|---|---|---|
| Nicotinamide adenine dinucleotide phosphate tetrasodium salt (NADPH) | 98% | 50650124 | Rocheroche Diagnostics GmbH |
| Phosphate buffer salt | / | SLBX1022 | SIGMA |
| Magnesium chloride hexahydrate ($MgCl_2 \cdot 6H_2O$) | ≥99.0% | BCBW0417 | SIGMA-ALDRICH |
| Formic acid (FA) | ≥96.0% | SHBJ0951 | SIGMA-ALDRICH |
| Acetonitrile | / | JA104130 | Merck KGaA |
| Methanol | / | I10099407026 | Merck KGaA |
| Ultra-pure water | / | / | Prepared by MilliQ water purification system |

2. Instruments

| Name | Model | Manufacturer |
|---|---|---|
| Chromatographic column Agilent | 2.1×150 mm, 3.5 | Agilent |
| Quadrupole high-resolution mass | Q Exactive | Thermoscientific |
| Ultra-high performance liquid | HCLass | Waters |
| Electric thermostatic oscillating water | DKZ | Shanghai Yiheng Technology |
| High-speed benchtop centrifuge | XIR | Thermo |
| Centrifuge | Legend Micro 21R | Thermo |
| Ultra-pure water system | Advantage A10 | Millipore |
| Electronic balance | XS105 | METTLER TOLEDO |

3. Test method

a. Preparation of phosphate buffer: The phosphate buffer salt powder was dissolved in 100 mL of purified water to prepare a 100 mM phosphate buffer solution with a pH of 7.4, which was then stored in a refrigerator at 4 °C for later use.

b. The test sample and the control sample testosterone were separately dissolved in DMSO to give 10 mM stock solutions. The 10 mM stock solutions were diluted to 100 μM with acetonitrile to serve as working solutions for later use.

c. Preparation of liver microsome working solutions: Mouse, rat, and human liver microsome stock solutions (at a concentration of 20 mg/mL) were separately taken and diluted to 0.56 mg/mL with the 100 mM phosphate buffer solution to serve as liver microsome working solutions.

d. Preparation of solutions: An appropriate amount of $MgCl_2$ was weighed and dissolved in the 100 mM phosphate buffer solution to prepare a 60 mM $MgCl_2$ solution. An appropriate amount of NADPH was weighed and dissolved in the 100 mM phosphate buffer solution to prepare a 20 mM NADPH solution, and then an equal volume of the 60 mM $MgCl_2$ solution was added to prepare an NADPH working solution containing 10 mM NADPH and 30 mM $MgCl_2$. An acetonitrile solution containing 20 ng/mL tolbutamide was prepared as a stop solution.

e. Liver microsome incubation: 2 μL of the compound working solution or the positive drug testosterone working solution was added to 178 μL of the liver microsome working solution (0.56 mg/mL). The mixture was gently and well mixed, and then pre-incubated in a shaking constant-temperature water bath at 37 °C for 10 min. Then, 20 μL of the NADPH working solution was added, and the resulting mixture was placed in the shaking constant-temperature water bath at 37 °C for timed incubation for 5 min, 10 min, 20 min, 30 min, and 60 min.

f. Termination of reaction: At the end of the corresponding incubation time, 400 μL of the stop solution was added to

terminate the reaction, and then the sample was immediately shaken for 1 min, followed by centrifugation at 13500 rpm for 10 min at 10 °C. After the centrifugation, the supernatant was removed, and diluted with water as required for each compound before LC-MS analysis. For the 0-minute sample, the stop solution was added first and well mixed with the liver microsomes, followed by the addition of the NADPH working solution. For the negative control sample, 20 μL of the 30 mM $MgCl_2$ solution was added instead of the NADPH working solution.

4. Data analysis:

$T_{1/2}$ and $CL_{int(mic)}$ were calculated using the following formulas:

$$C_t = C_0 \times e^{-k_e \cdot t}$$

When

$$C_t = 1/2\ C_0,\ T_{1/2} = \ln 2/k_e = 0.693/k_e$$

$$CL_{int(mic)} = 0.693/T_{1/2}/\text{incubated liver microsomal protein concentration (mg/mL)}$$

$$CL_{int(liver)} = CL_{int(mic)} \times \text{liver microsomal protein (mg)/liver weight (g)} \times \text{liver-to-body weight ratio}$$

Liver microsomal protein (mg)/liver weight (g): The value was 45 in both animal and human species.
Liver-to-body weight ratio: The parameters in mice, rats, and humans were 88 g/kg, 40 g/kg, and 20 g/kg, respectively.

5. Test results: The metabolic stability results of the compound of the present disclosure in mouse, rat, and human liver microsomes are shown in Table 13.

Table 13. Metabolic stability results in mouse, rat, and human liver microsomes

| Compound | Species | Remaining amount at 60 min | $T_{1/2}$ | CLint(mic) | $CL_{int(liver)}$ | Clearance rate |
|---|---|---|---|---|---|---|
| | | % | min | μL/min/mg protein | mL/min/kg | |
| Formula I | Mouse | 19.2 | 26.7 | 52.0 | 206 | Medium |
| | Rat | 3.06 | 12.2 | 113 | 204 | High |
| | Human | 26.0 | 30.8 | 45.0 | 40.5 | Medium |

**Test Example 5. Pharmacodynamic Study of Compound of Formula I in Mice**

1. Test materials:

Balb/C Nude mice, SPF grade, male, weighing 18-20 g, purchased from Jiangsu GemPharmatech Co., Ltd.
HCT116 cells, purchased from ATCC.
Matrigel Matrix, Cat. No. 354248, purchased from Becton, Dickinson and Company.
Kolliphor® HS 15, Cat. No. 42966, purchased from Sigma-Aldrich.

2. Test method:

a. Establishment of tumor-bearing mouse model: HCT116 cells in the logarithmic growth phase were cultured, collected, and counted. The cells were then subcutaneously inoculated into Balb/C Nude mice at $1 \times 10^6$ cells/mouse. The tumor volume and body weight of the mice were measured regularly. Seven days after inoculation, mice with a tumor volume of 150 $mm^3$ were selected and randomly grouped for corresponding drug treatment.

b. Animal grouping and administration: All mice were randomly grouped, including 6 mice in a negative control group (sham group, 20% Solutol HS15), 6 mice in an onvansertib monotherapy group (ONV-20 mg/kg group), and 6 mice in a formula I compound monotherapy group (T1-20 mg/kg group). The mice were subjected to intragastric administration once daily for 21 consecutive days, and the tumor volume and body weight of the mice were measured regularly.

3. Data processing: All data were organized in EXCEL; One-way ANOVA analysis and plotting were performed using GraphPad Prism 8.0 software. When *p < 0.05,* it was considered statistically significant.

$$\text{Tumor volume(V)} = 0.5 \times \text{Long diameter} \times \text{Short diameter}^2$$

$$\text{Tumor growth inhibition rate(TGI, \%)} = \frac{\text{Mean tumor volume of negative control group} - \text{Mean tumor volume of drug treatment group}}{\text{Mean tumor volume of negative control group}} \times 100\%$$

4. Test results:

As shown in FIG. 11, after 11 days of drug treatment in the mice, compared to the sham group, the T1-20 mg/kg group achieved a tumor growth inhibition rate of 82.0% ($p < 0.0001$), and the ONV-20 mg/kg group exhibited a tumor growth inhibition rate of 54.9% ($p < 0.05$). After 16 consecutive days of administration, compared to the sham group, the T1-20 mg/kg group achieved a tumor growth inhibition rate of 86.5% ($p < 0.001$), and the ONV-20 mg/kg group exhibited a tumor growth inhibition rate of 39.6% ($p < 0.05$). Furthermore, the data between the T1-20 mg/kg and ONV-20 mg/kg groups showed a significant difference ($p < 0.01$). After 21 consecutive days of administration, compared to the sham group, the T1-20 mg/kg group achieved a tumor growth inhibition rate of 88.3% ($p < 0.0001$), and the ONV-20 mg/kg group exhibited a tumor growth inhibition rate of 37.0% ($p < 0.05$). Furthermore, the data between the T1-20 mg/kg and ONV-20 mg/kg groups showed a significant difference ($p < 0.01$).

As shown in FIG. 12, during the experimental period, there was no significant difference in body weight measured each time among the groups of animals.

5. Experimental conclusion: The compound of formula I of the present disclosure had a significant inhibitory effect on the growth of colorectal tumor volume, and its efficacy was superior to that of the control drug onvansertib at the same dose.

**Test Example 6: Hygroscopicity Study**

The hygroscopicity of the crystal form A, the crystal form A of the maleate, the crystal form A of the fumarate, and the crystal form A of the succinate of the compound of formula I was evaluated using DVS at 25 °C. The four samples were all anhydrous crystal forms. Before testing, the samples were equilibrated at 0% RH to remove water or solvent adsorbed on the surface.

The moisture adsorption values of the crystal form A, the crystal form A of the maleate, the crystal form A of the fumarate, and the crystal form A of the succinate of the compound of formula I at 25 °C/80% RH were 0.78%, 1.43%, 0.86%, and 1.04%, respectively, indicating that the samples had slight hygroscopicity; no crystal form transformation occurred in any sample after DVS testing.

**Test Example 7: Solid State Stability Study**

The solid state stability of the crystal form A, the crystal form A of the maleate, the crystal form A of the fumarate, and the crystal form A of the succinate of the compound of formula I was evaluated under three conditions: 25 °C/60% RH, 40 °C/75% RH, and 60 °C. About 5 mg of a sample was weighed into an HPLC vial, and the uncovered vial was placed under the stability conditions. Samples were taken after one week and two weeks of storage for crystal form chemical purity testing by HPLC and crystal form detection by XRPD. The solid state stability results are shown in Table 14, indicating that after storage for two weeks under the stability conditions, the crystal forms of the crystal form A, the crystal form A of the maleate, the crystal form A of the fumarate, and the crystal form A of the succinate of the compound of formula I remained unchanged, and the purity of the samples showed no significant decrease.

Table 14. Stability data of crystal forms

| Crystal form | Initial purity (area%) | Conditions | 1 week | | 2 weeks | |
|---|---|---|---|---|---|---|
| | | | Purity (area%) | Crystal form change | Purity (area%) | Crystal form change |
| Crystal form A of compound of formula I | 98.33 | 25 °C/60% RH | 98.21 | No | 98.29 | No |
| | | 40 °C/75% RH | 98.26 | No | 98.27 | No |
| | | 60 °C | 98.18 | No | 98.30 | No |
| Crystal form A of maleate of compound of formula I | 98.38 | 25 °C/60% RH | 98.38 | No | 98.39 | No |
| | | 40 °C/75% RH | 98.38 | No | 98.37 | No |
| | | 60 °C | 98.34 | No | 98.39 | No |
| Crystal form A of fumarate of compound of formula I | 98.36 | 25 °C/60% RH | 98.36 | No | 98.36 | No |
| | | 40 °C/75% RH | 98.35 | No | 98.38 | No |
| | | 60 °C | 98.35 | No | 98.33 | No |
| Crystal form A of succinate of compound of formula I | 98.39 | 25 °C/60% RH | 98.39 | No | 98.39 | No |
| | | 40 °C/75% RH | 98.39 | No | 98.39 | No |
| | | 60 °C | 98.39 | No | 98.25 | No |

## Claims

1. A crystal form A of the compound of formula I, having, in an X-ray powder diffraction pattern using Cu-Kα radiation, characteristic diffraction peaks at the following 2θ angles: 4.807±0.2°, 8.532±0.2°, 11.645±0.2°, 15.160±0.2°, 18.347±0.2°, and 25.514±0.2°; preferably, the crystal form A has characteristic diffraction peaks at the following 2θ angles: 4.807±0.2°, 8.532±0.2°, 11.645±0.2°, 15.160±0.2°, 16.768±0.2°, 18.347±0.2°, 19.000±0.2°, 19.247 ±0.2°, and 25.514±0.2°; more preferably, the X-ray powder diffraction pattern using Cu-Kα radiation is as shown in FIG. 1-1,

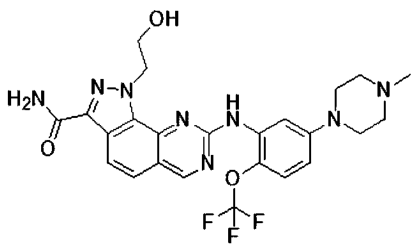

formula I .

2. A pharmaceutically acceptable salt of the compound of formula I, selected from the group consisting of a hydrochloride, sulfate, maleate, L-aspartate, L-tartrate, phosphate, fumarate, citrate, malate, glycolate, L-malate, hippurate, succinate, adipate, p-toluenesulfonate, methanesulfonate, benzenesulfonate, oxalate, malonate, gentisate, or hydrobromide; preferably, a maleate, fumarate, or succinate.

3. A crystal form A of a maleate of the compound of formula I, having, in an X-ray powder diffraction pattern using Cu-Kα radiation, characteristic diffraction peaks at the following 2θ angles: 8.455±0.2°, 12.802±0.2°, 13.894±0.2°, 19.029 ±0.2°, 20.917±0.2°, and 21.383±0.2°; preferably, the crystal form A has characteristic diffraction peaks at the following 2θ angles: 8.455±0.2°, 12.313±0.2°, 12.802±0.2°, 13.894±0.2°, 17.051±0.2°, 17.713±0.2°, 19.029 ±0.2°, 20.917±0.2°, 21.383±0.2°, and 24.787±0.2°; more preferably, the crystal form A has characteristic diffraction peaks at the following 2θ angles: 6.883±0.2°, 8.455±0.2°, 12.313±0.2°, 12.802±0.2°, 13.894±0.2°, 14.640±0.2°, 17.051±0.2°, 17.369±0.2°, 17.713±0.2°, 19.029±0.2°, 20.917±0.2°, 21.383±0.2°, 23.818±0.2°, 24.787±0.2°, and 25.823±0.2°; most preferably, the X-ray powder diffraction pattern using Cu-Kα radiation is as shown in FIG. 2-1.

**4.** A crystal form A of a fumarate of the compound of formula I, having, in an X-ray powder diffraction pattern using Cu-Kα radiation, characteristic diffraction peaks at the following 2θ angles: 7.931±0.2°, 15.752±0.2°, 16.012±0.2°, 20.426 ±0.2°, 21.636±0.2°, 22.902±0.2°, and 24.234±0.2°; preferably, the crystal form A has characteristic diffraction peaks at the following 20 angles: 7.931±0.2°, 12.635±0.2°, 13.761±0.2°, 15.752±0.2°, 16.012±0.2°, 16.972±0.2°, 19.481±0.2°, 20.426±0.2°, 21.636±0.2°, 22.285±0.2°, 22.641±0.2°, 22.902±0.2°, 23.597±0.2°, 24.234±0.2°, 27.948±0.2°, and 29.329±0.2°; more preferably, the X-ray powder diffraction pattern using Cu-Kα radiation is as shown in FIG. 3-1.

**5.** A crystal form A of a succinate of the compound of formula I, having, in an X-ray powder diffraction pattern using Cu-Kα radiation, characteristic diffraction peaks at the following 2θ angles: 7.771±0.2°, 13.539±0.2°, 15.519±0.2°, 20.105 ±0.2°, 21.347±0.2°, and 22.552±0.2°; preferably, the crystal form A has characteristic diffraction peaks at the following 2θ angles: 6.655±0.2°, 7.771±0.2°, 13.539±0.2°, 15.519±0.2°, 15.781±0.2°, 17.709±0.2°, 20.105±0.2°, 21.347±0.2°, 22.552±0.2°, and 23.887±0.2°; more preferably, the X-ray powder diffraction pattern using Cu-Kα radiation is as shown in FIG. 4-1.

**6.** A crystal form B of a hemifumarate of the compound of formula I, having, in an X-ray powder diffraction pattern using Cu-Kα radiation, characteristic diffraction peaks at the following 2θ angles: 15.084±0.2°, 15.501±0.2°, 17.311±0.2°, 24.838±0.2°, 26.132±0.2°, and 26.736±0.2°; preferably, the crystal form B has characteristic diffraction peaks at the following 2θ angles: 8.570±0.2°, 12.545±0.2°, 15.084±0.2°, 15.501±0.2°, 17.311±0.2°, 24.838±0.2°, 26.132 ±0.2°, and 26.736±0.2°; more preferably, the X-ray powder diffraction pattern using Cu-Kα radiation is as shown in FIG. 5-1.

**7.** A crystal form C of a hemifumarate of the compound of formula I, having, in an X-ray powder diffraction pattern using Cu-Kα radiation, characteristic diffraction peaks at the following 2θ angles: 7.553±0.2°, 12.716±0.2°, 13.547±0.2°, 15.313±0.2°, 19.089±0.2°, and 22.755±0.2°; preferably, the crystal form C has characteristic diffraction peaks at the following 2θ angles: 6.637±0.2°, 7.553±0.2°, 12.716±0.2°, 13.547±0.2°, 15.313±0.2°, 19.089±0.2°, 20.121±0.2°, 20.609±0.2°, and 22.755±0.2°; more preferably, the X-ray powder diffraction pattern using Cu-Kα radiation is as shown in FIG. 6-1.

**8.** A crystal form D of a hemifumarate of the compound of formula I, having, in an X-ray powder diffraction pattern using Cu-Kα radiation, characteristic diffraction peaks at the following 20 angles: 6.881±0.2°, 8.103±0.2°, 12.572±0.2°, and 13.831±0.2°; preferably, the crystal form D has characteristic diffraction peaks at the following 2θ angles: 6.881 ±0.2°, 8.103±0.2°, 12.572±0.2°, 13.831±0.2°, 19.569±0.2°, 20.533±0.2°, and 24.021±0.2°; more preferably, the X-ray powder diffraction pattern using Cu-Kα radiation is as shown in FIG. 7-1.

**9.** A crystal form E of a hemifumarate of the compound of formula I, having, in an X-ray powder diffraction pattern using Cu-Kα radiation, characteristic diffraction peaks at the following 20 angles: 6.188±0.2°, 12.492±0.2°, 15.473±0.2°, 17.729±0.2°, 25.017±0.2°, and 26.603±0.2°; preferably, the crystal form E has characteristic diffraction peaks at the following 2θ angles: 6.188±0.2°, 12.492±0.2°, 15.473±0.2°, 17.729±0.2°, 25.017±0.2°, 25.275±0.2°, 26.603 ±0.2°, and 31.795±0.2°; more preferably, the X-ray powder diffraction pattern using Cu-Kα radiation is as shown in FIG. 8-1.

**10.** A crystal form F of a hemifumarate of the compound of formula I, having, in an X-ray powder diffraction pattern using Cu-Kα radiation, characteristic diffraction peaks at the following 20 angles: 8.866±0.2°, 16.651±0.2°, 25.199±0.2°, and 26.157±0.2°; preferably, the crystal form F has characteristic diffraction peaks at the following 2θ angles: 8.185 ±0.2°, 8.866±0.2°, 13.449±0.2°, 16.651±0.2°, 16.841±0.2°, 25.199±0.2°, and 26.157±0.2°; more preferably, the X-ray powder diffraction pattern using Cu-Kα radiation is as shown in FIG. 9-1.

**11.** A crystal form G of a hemifumarate of the compound of formula I, having, in an X-ray powder diffraction pattern using Cu-Kα radiation, characteristic diffraction peaks at the following 20 angles: 8.925±0.2°, 12.025±0.2°, 12.948±0.2°, 13.525±0.2°, 15.763±0.2°, 16.793±0.2°, 21.133±0.2°, 23.547±0.2°, 25.164±0.2°, and 26.101±0.2°; preferably, the crystal form G has characteristic diffraction peaks at the following 2θ angles: 6.707±0.2°, 7.294±0.2°, 8.925 ±0.2°, 12.025±0.2°, 12.948±0.2°, 13.525±0.2°, 15.510±0.2°, 15.763±0.2°, 16.417±0.2°, 16.793±0.2°, 18.923 ±0.2°, 21.133±0.2°, 23.547±0.2°, 25.164±0.2°, and 26.101±0.2°; more preferably, the X-ray powder diffraction pattern using Cu-Kα radiation is as shown in FIG. 10-1.

**12.** A pharmaceutical composition, comprising the crystal form according to any one of claims 1 and 3-11 or the pharmaceutically acceptable salt according to claim 2, and a pharmaceutically acceptable carrier.

**13.** Use of the crystal form according to any one of claims 1 and 3-11, the pharmaceutically acceptable salt according to claim 2, or the pharmaceutical composition according to claim 12 in the preparation of a medicament for preventing and/or treating a disease caused by and/or associated with dysregulation of protein kinase activity.

**14.** Use of the crystal form according to any one of claims 1 and 3-11, the pharmaceutically acceptable salt according to claim 2, or the pharmaceutical composition according to claim 12 in the preparation of a medicament for preventing or treating a disease caused by and/or associated with dysregulation of PLK1 kinase activity.

**15.** The use according to claim 12 or 14, wherein the disease comprises a cancer, a cell proliferative disease, a viral infection, an autoimmune disease, and a neurodegenerative disease.

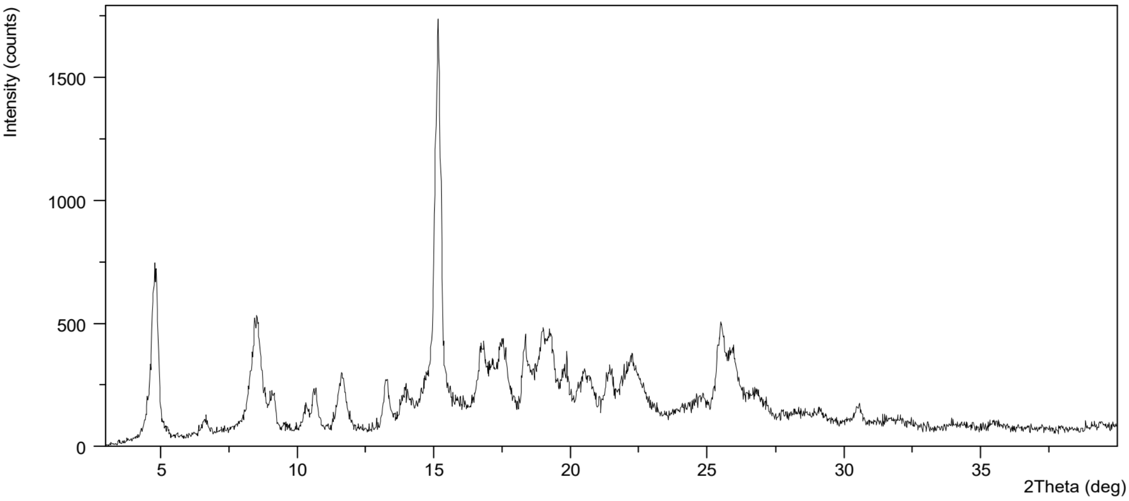


FIG. 1-1

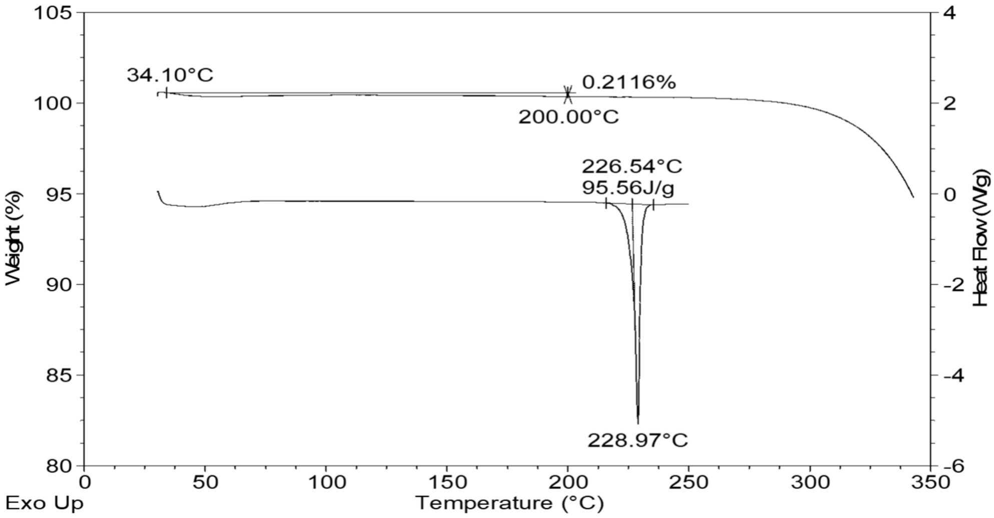


FIG. 1-2

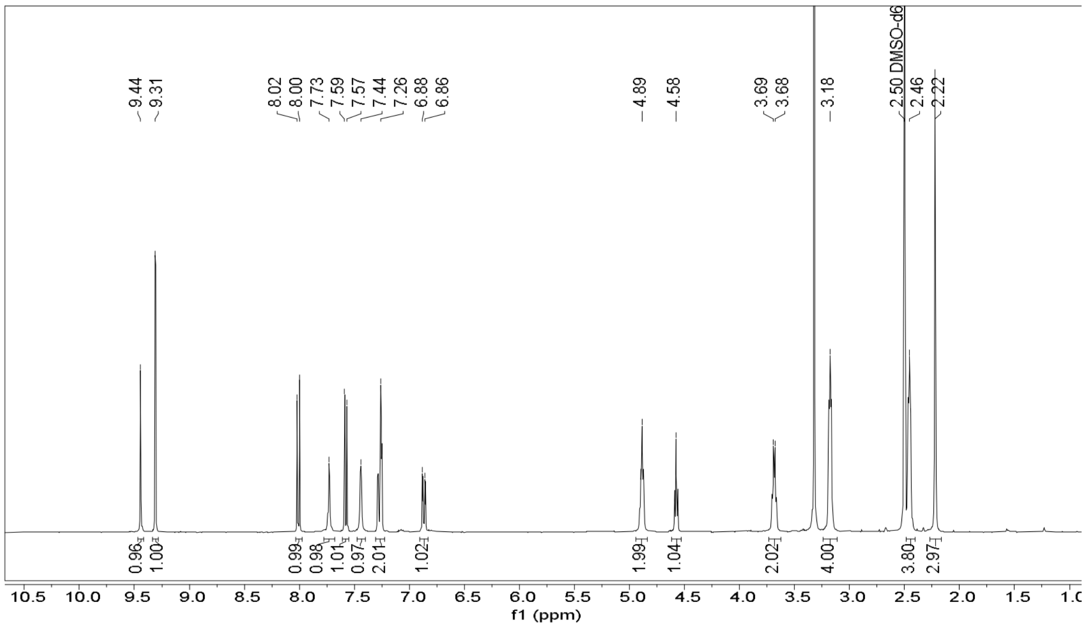


FIG. 1-3

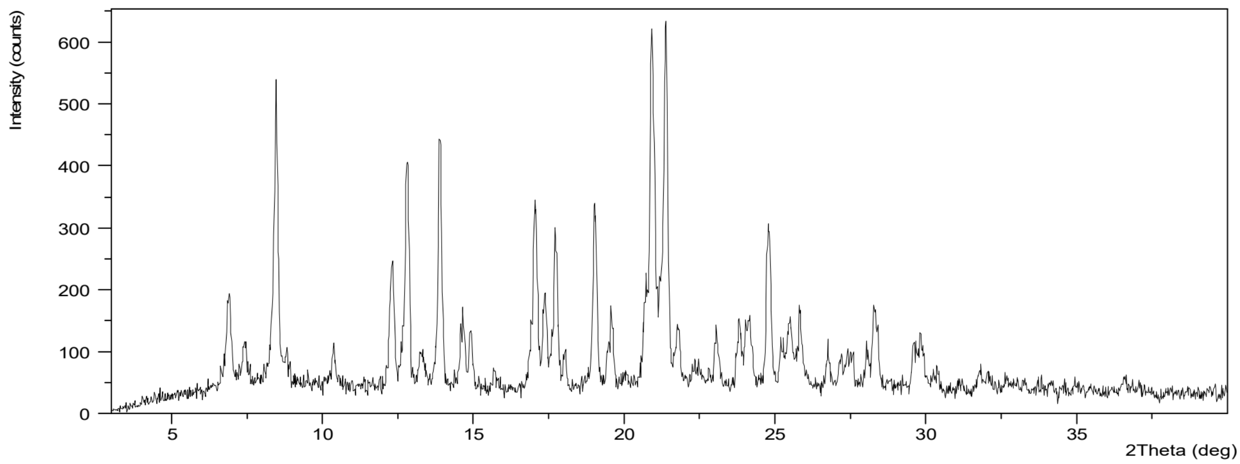


FIG. 2-1

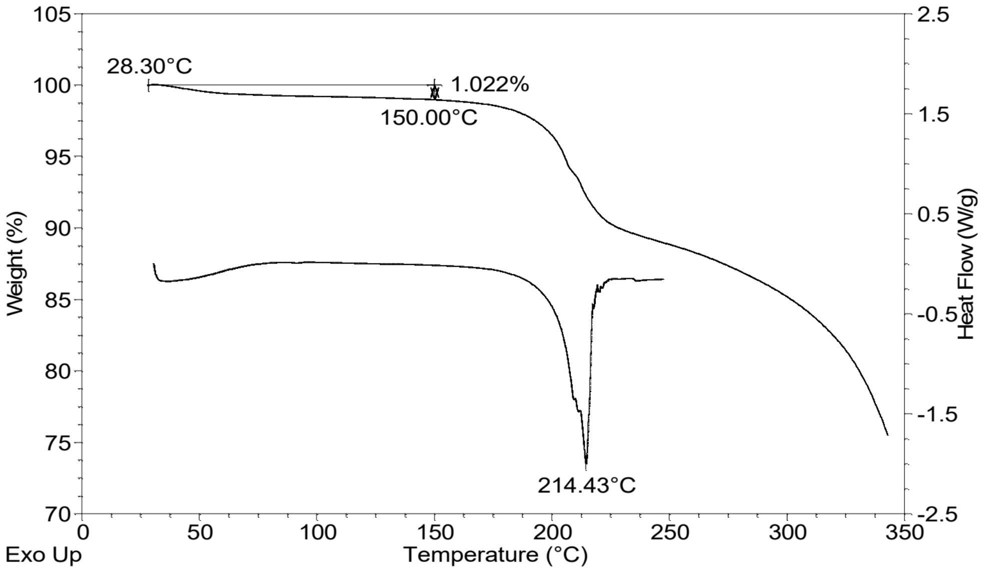


FIG. 2-2

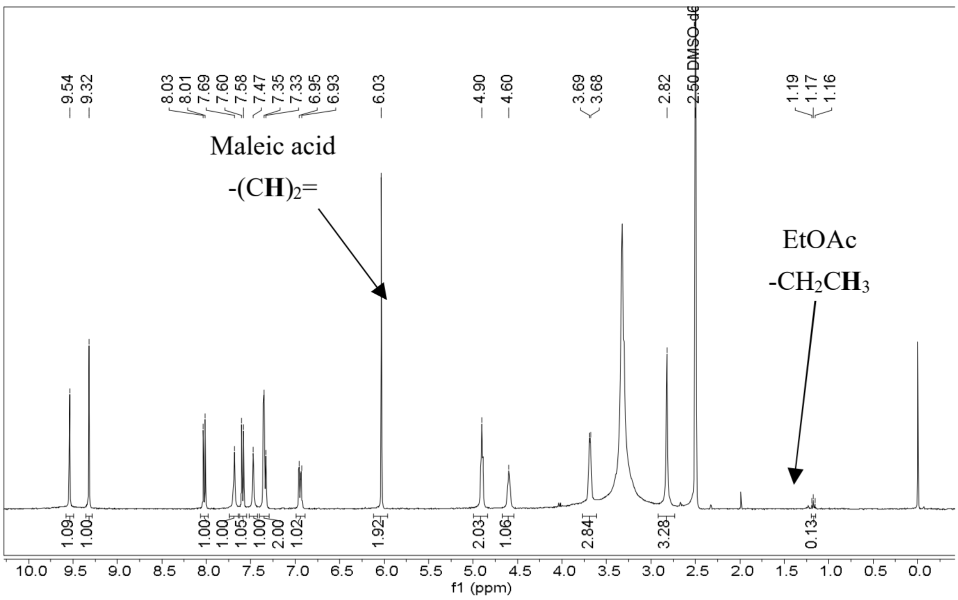


FIG. 2-3

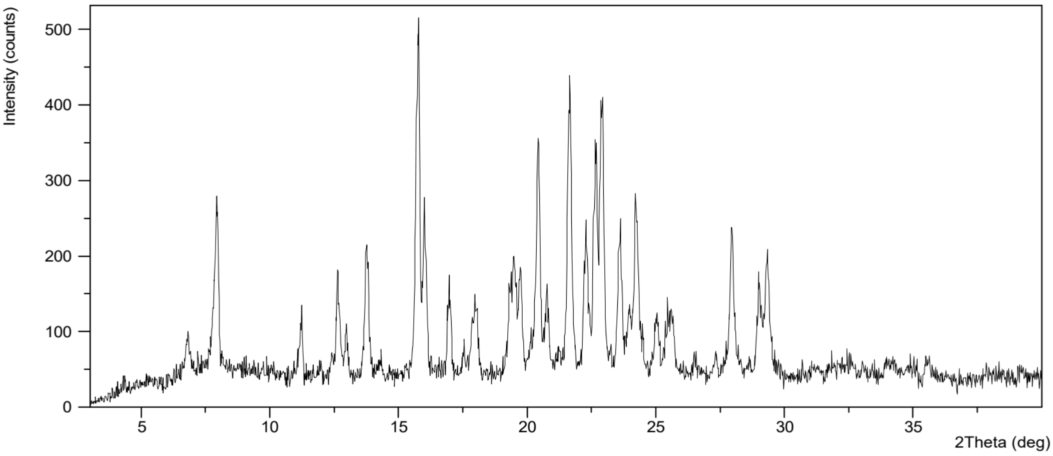


FIG. 3-1

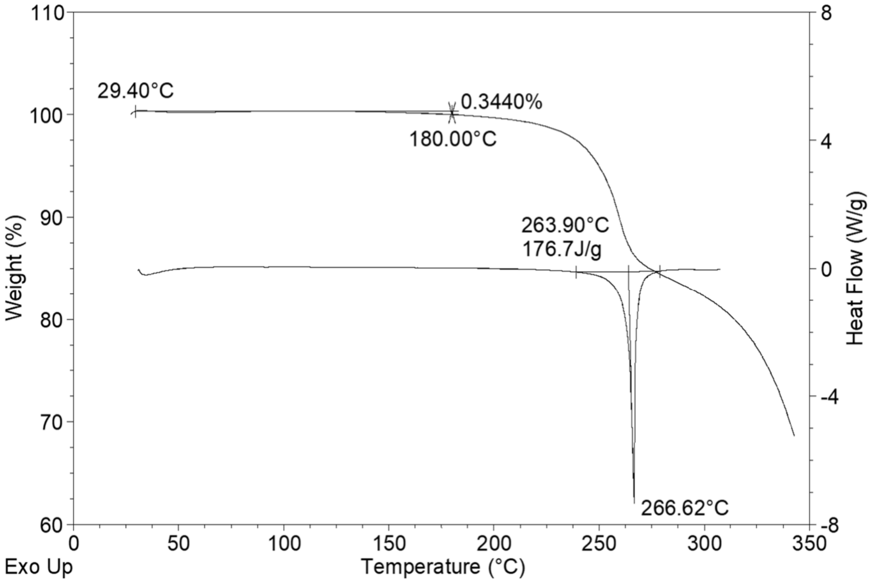


FIG. 3-2

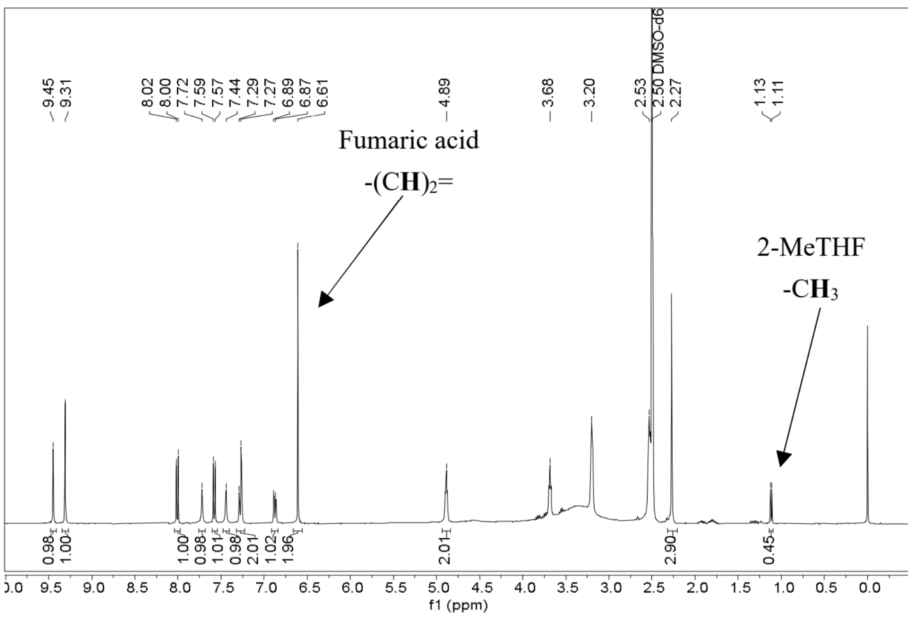


FIG. 3-3

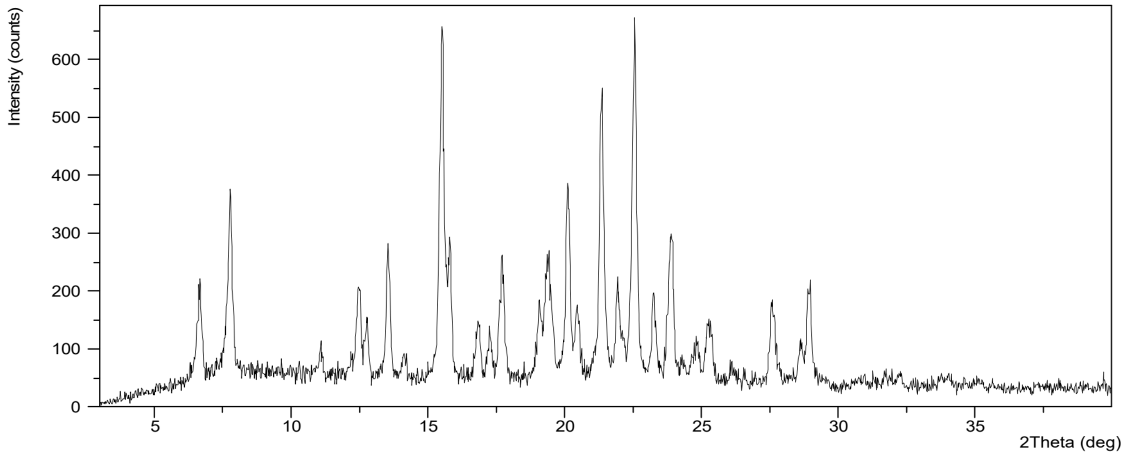


FIG. 4-1

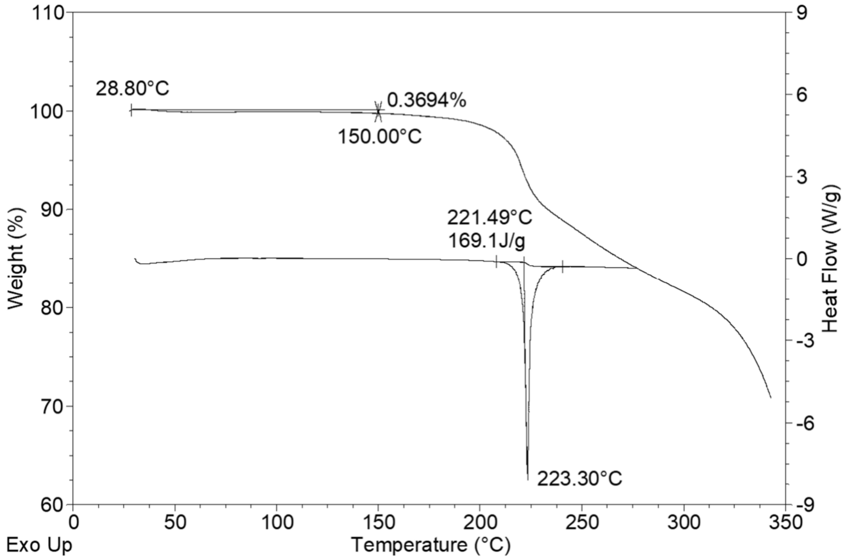


FIG. 4-2

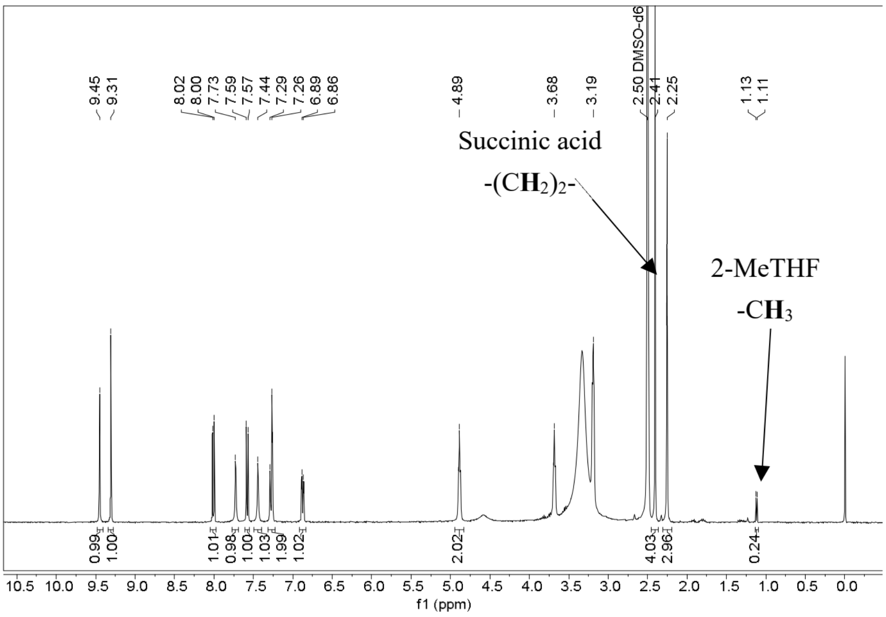


FIG. 4-3

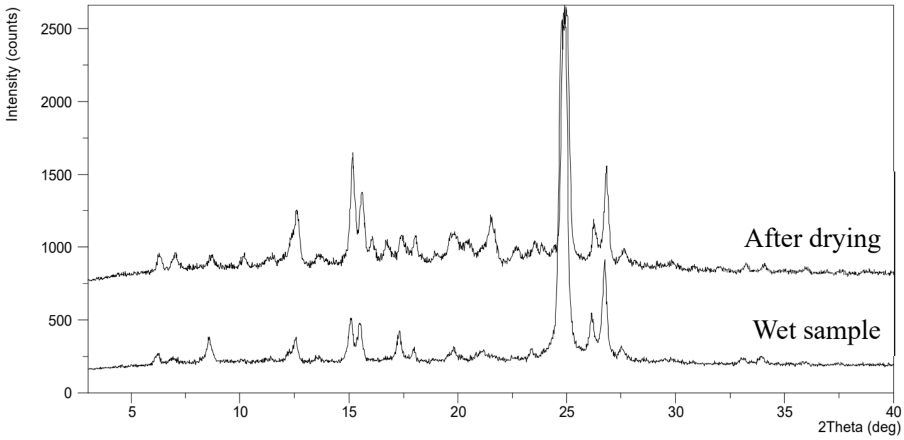


FIG. 5-1

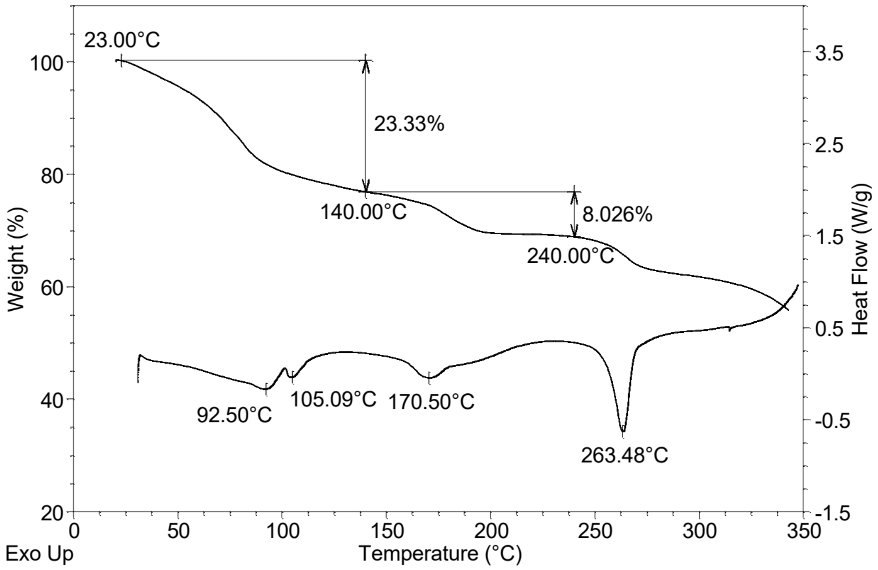


FIG. 5-2

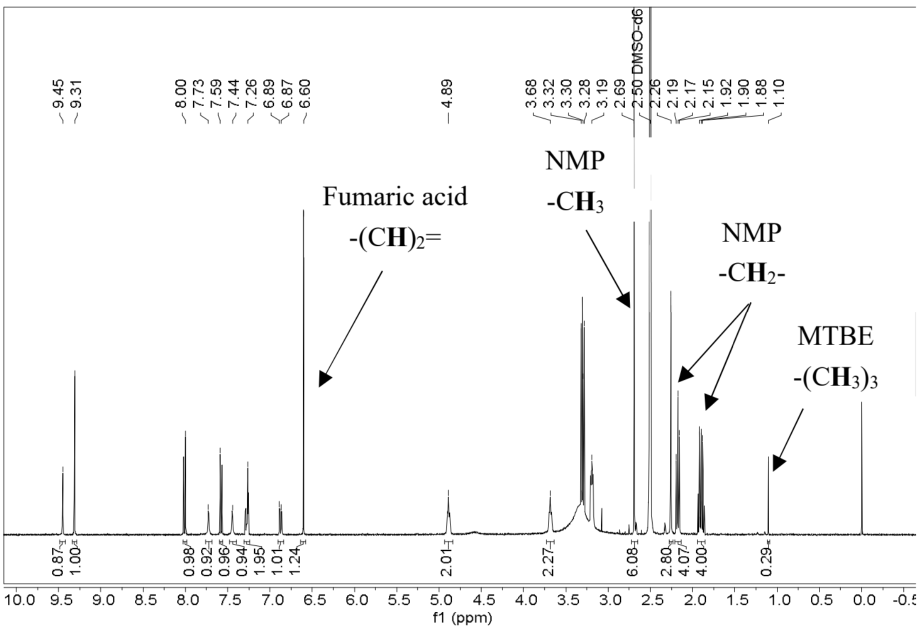


FIG. 5-3

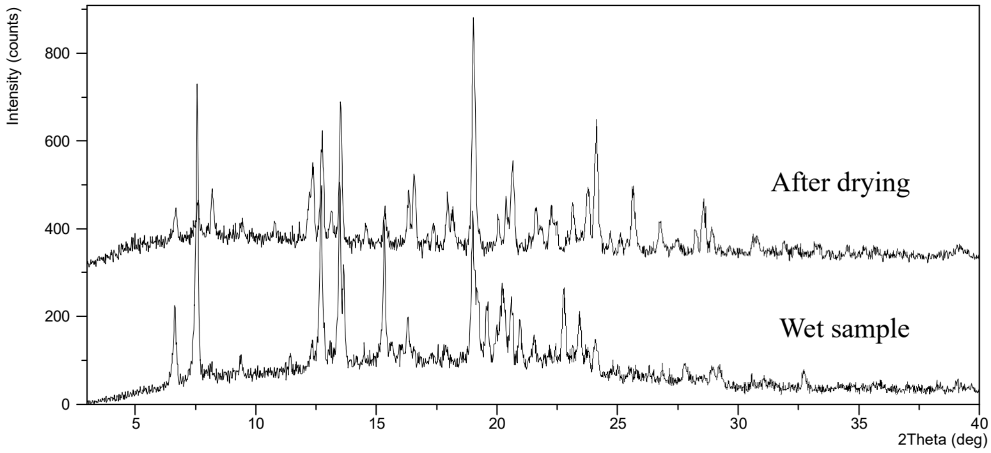


FIG. 6-1

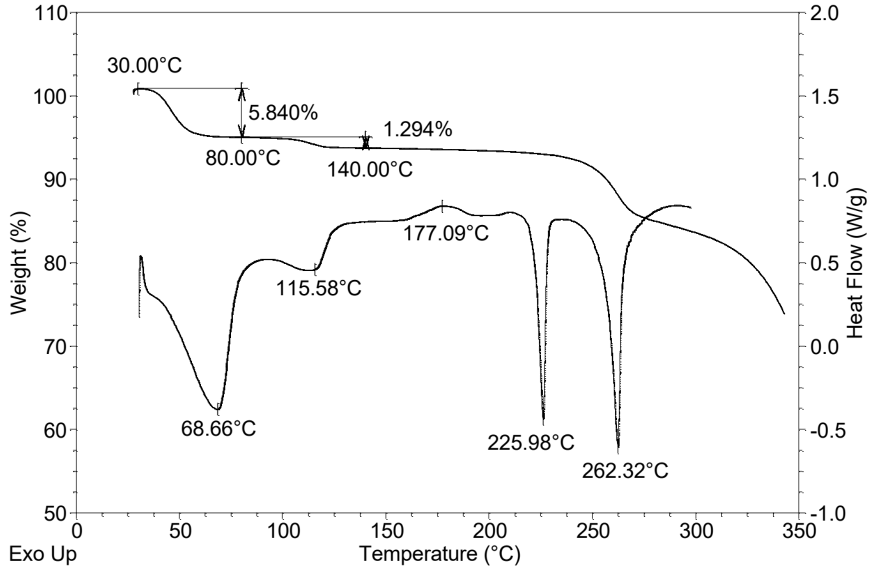


FIG. 6-2

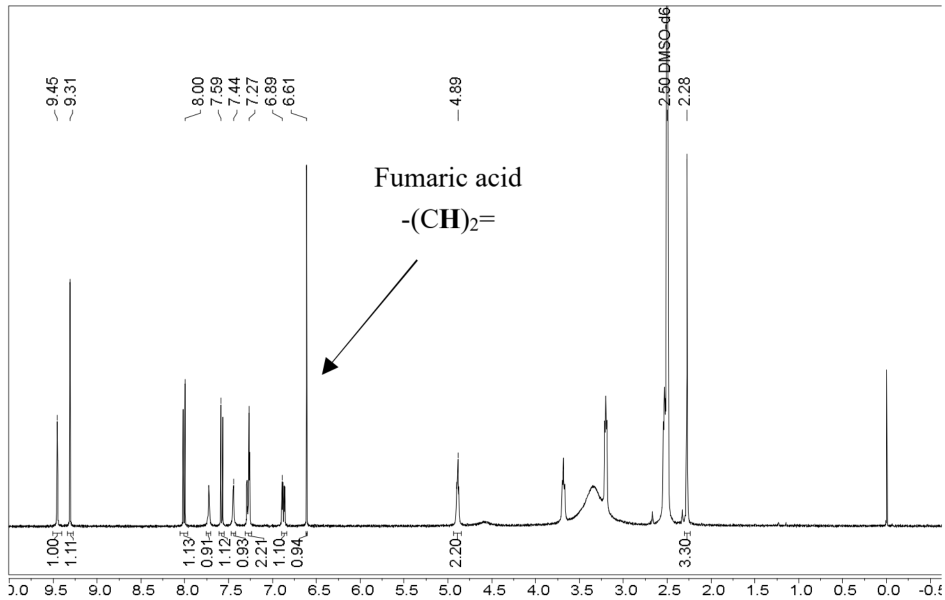


FIG. 6-3

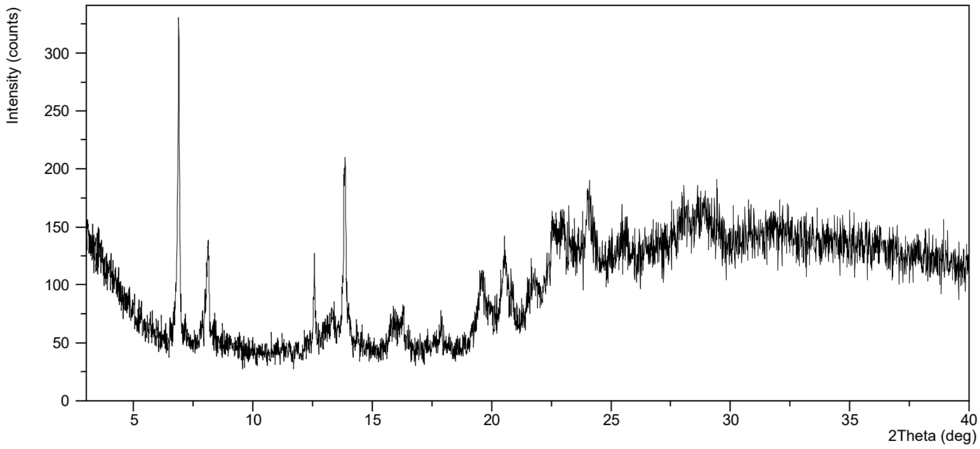


FIG. 7-1

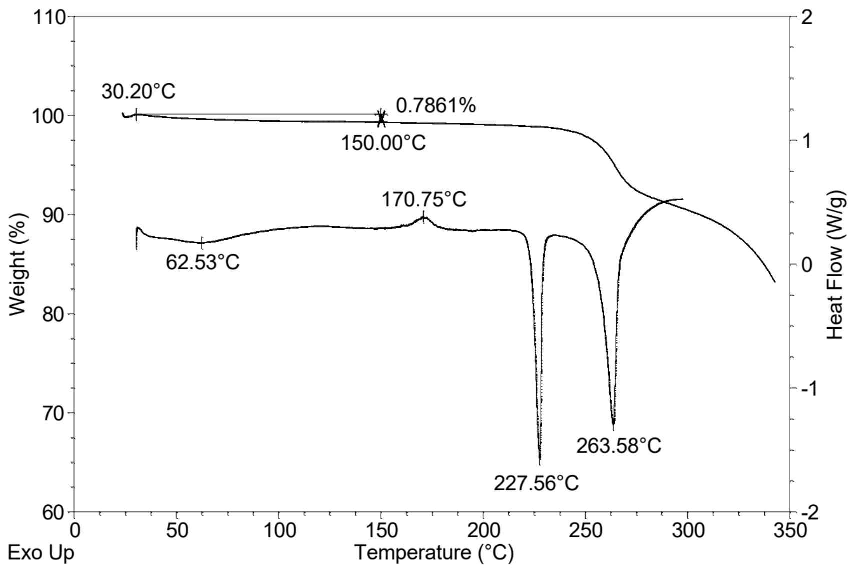


FIG. 7-2

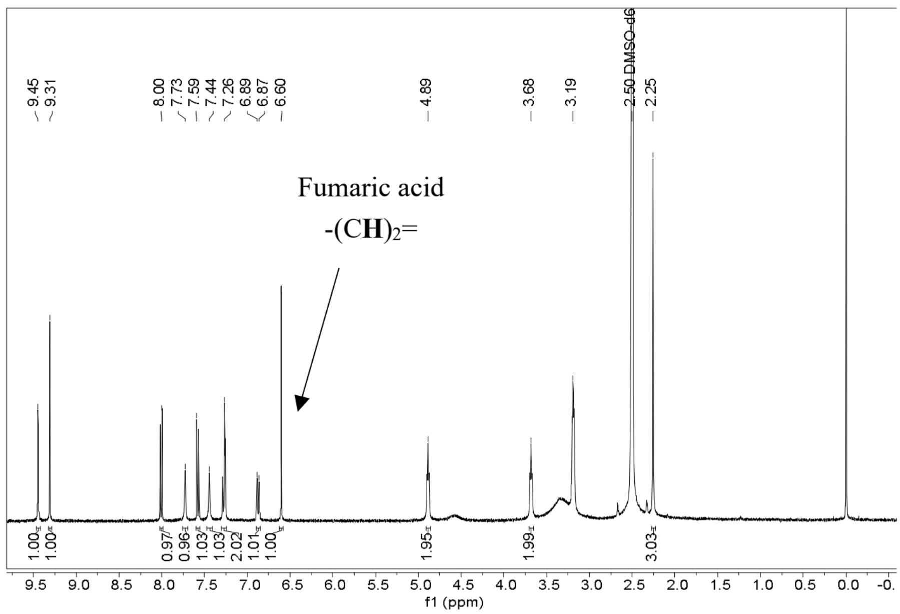


FIG. 7-3

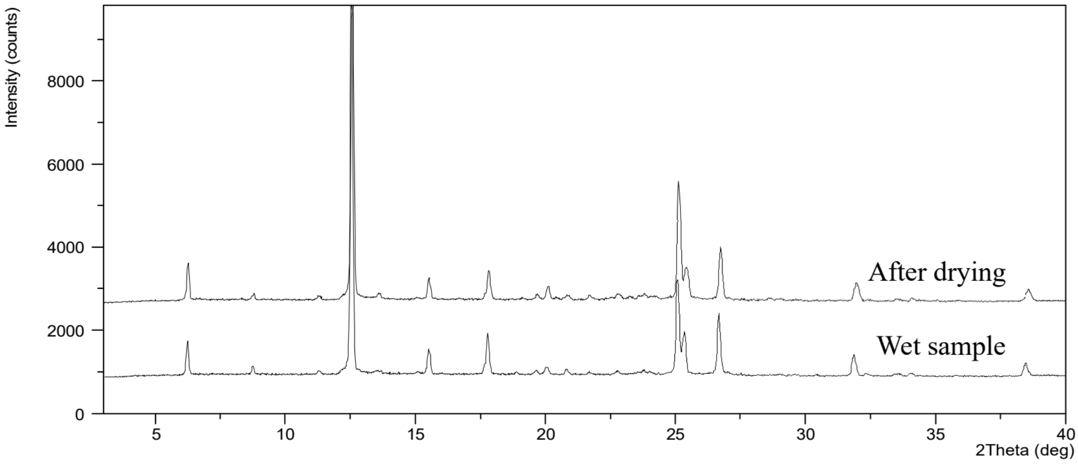


FIG. 8-1

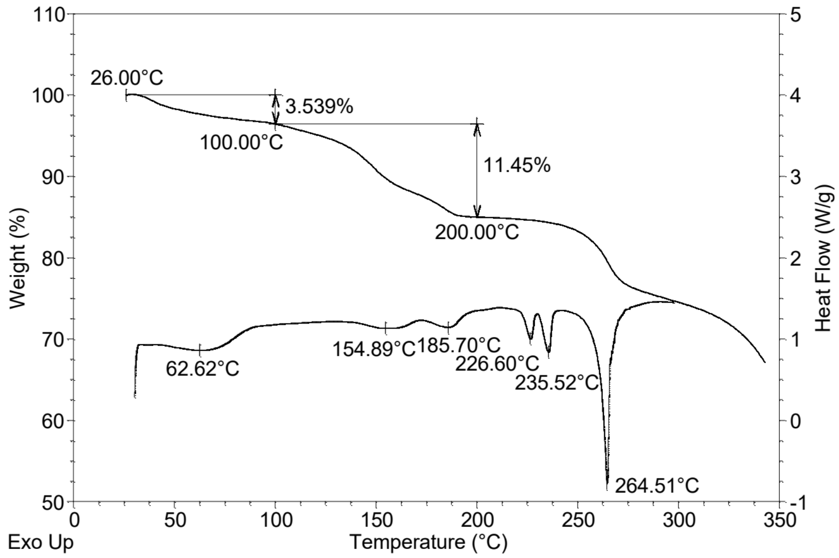


FIG. 8-2

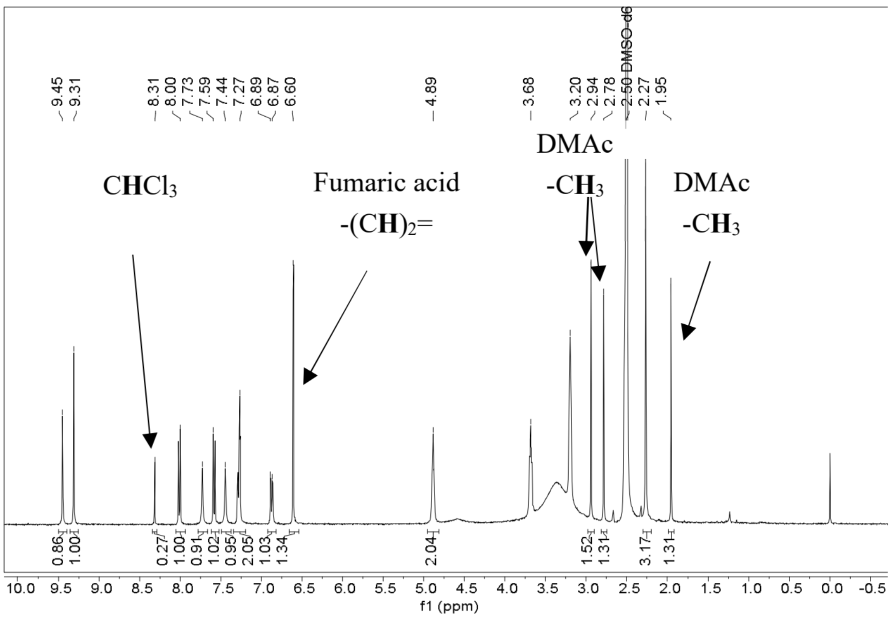


FIG. 8-3

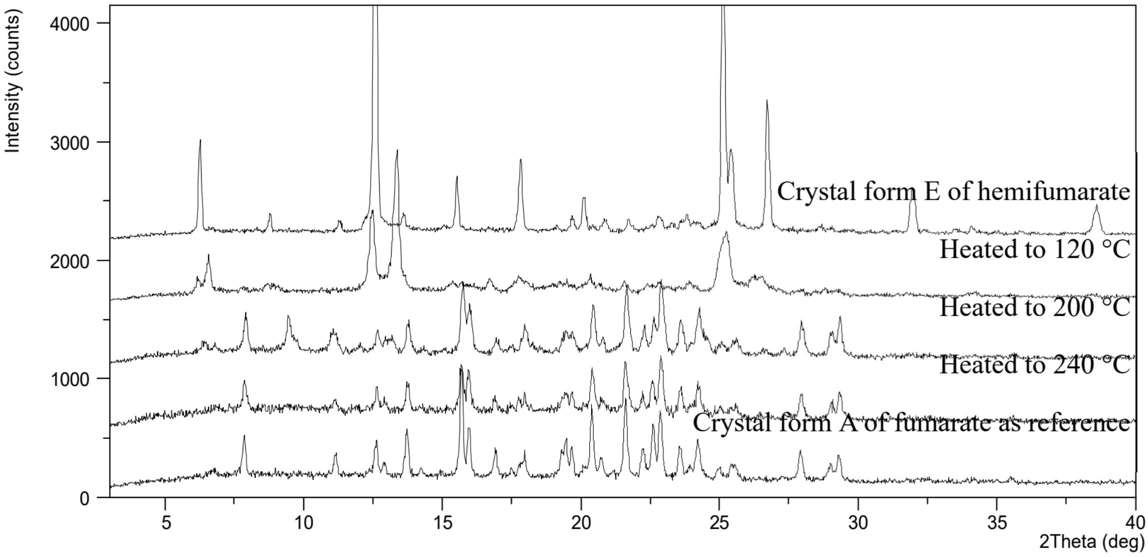


FIG. 8-4

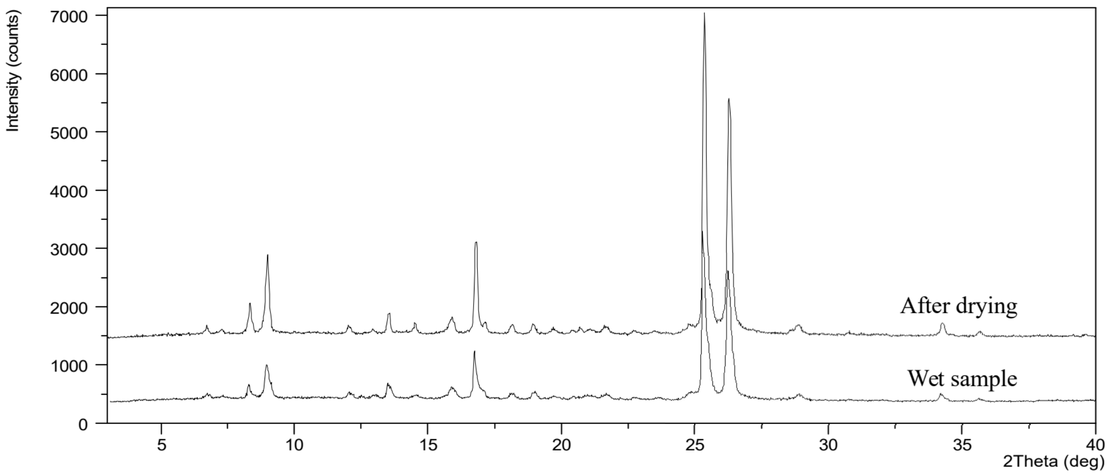


FIG. 9-1

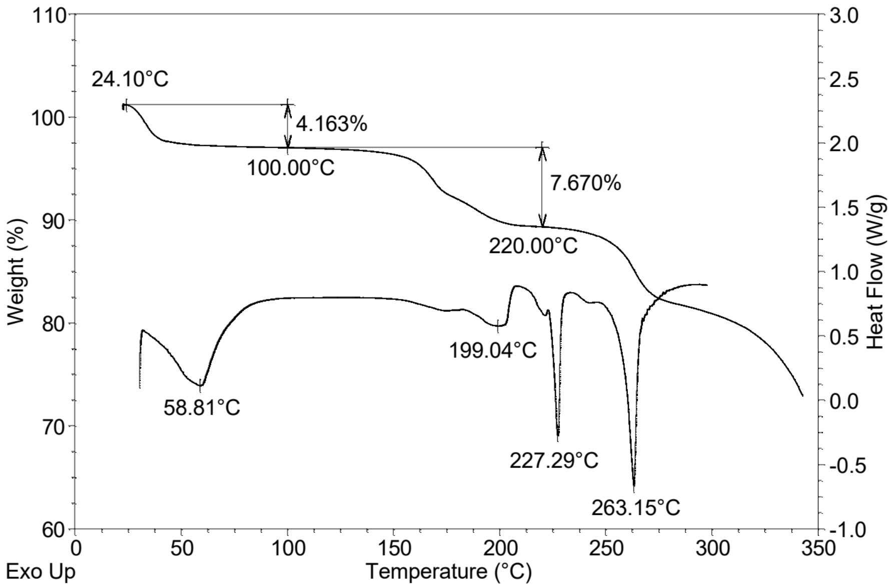


FIG. 9-2

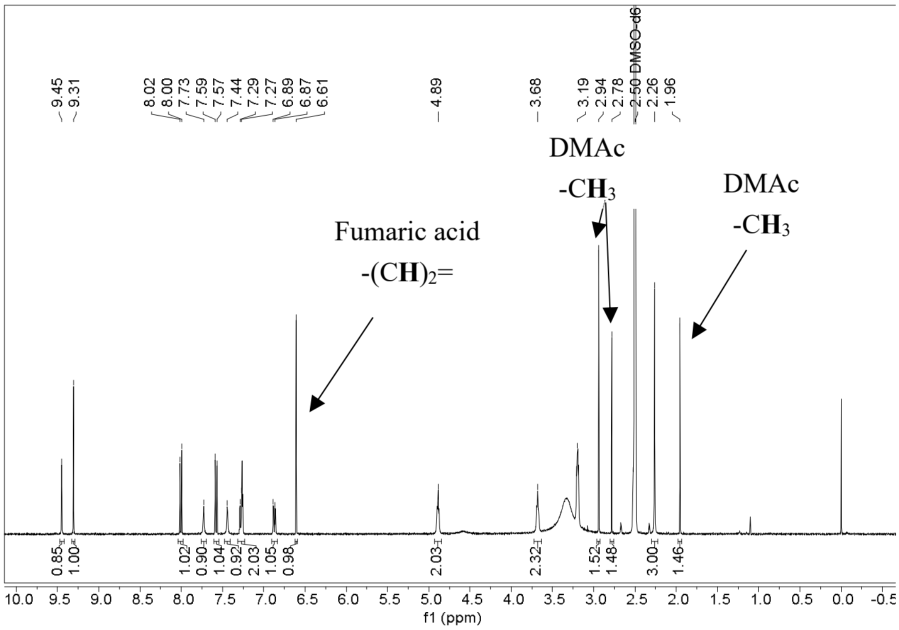


FIG. 9-3

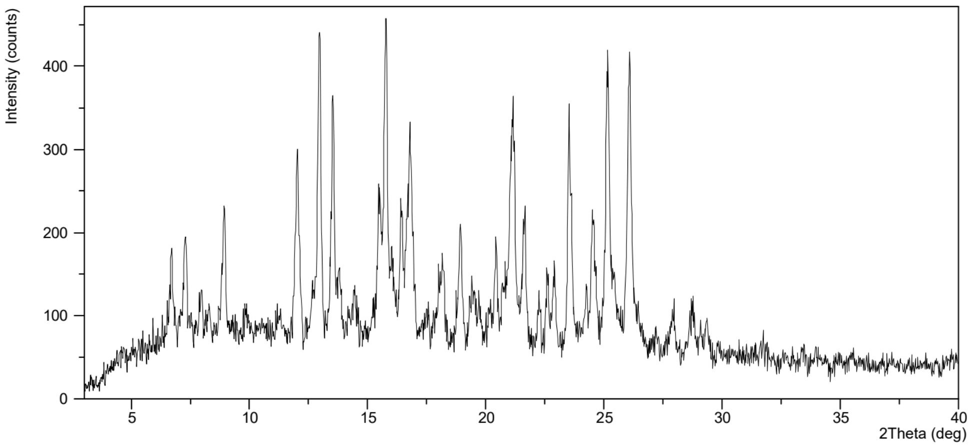


FIG. 10-1

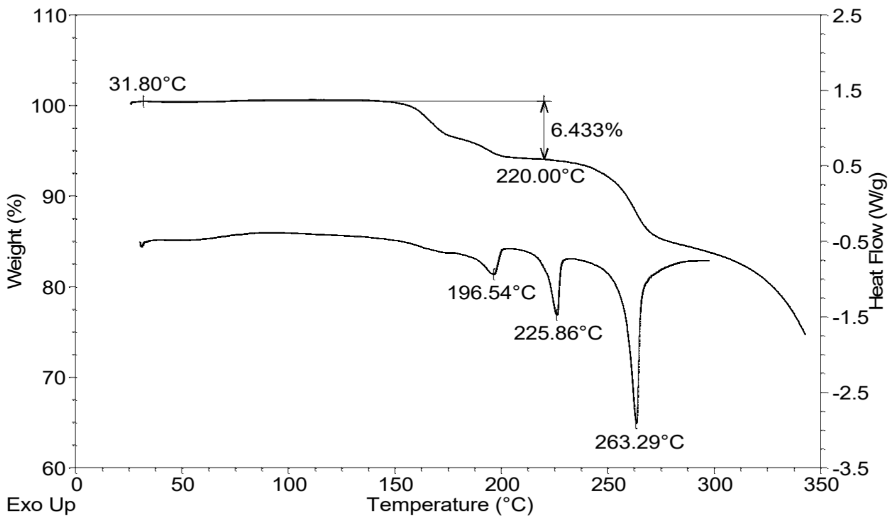


FIG. 10-2

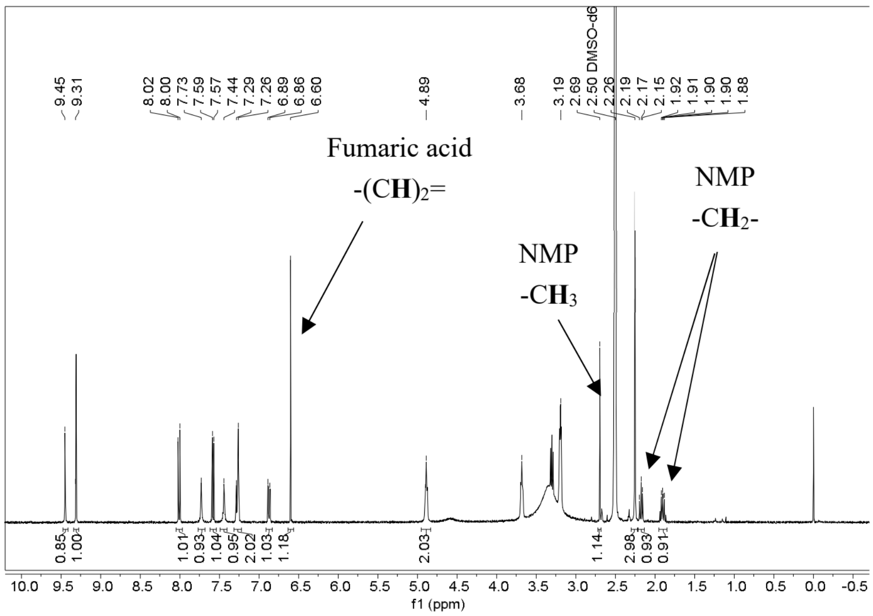


FIG. 10-3

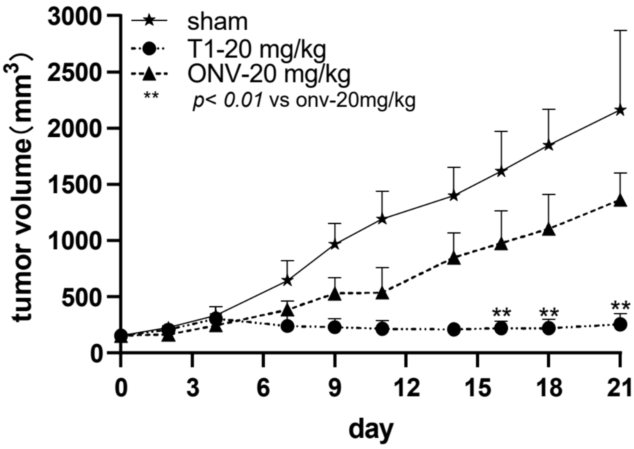


FIG. 11

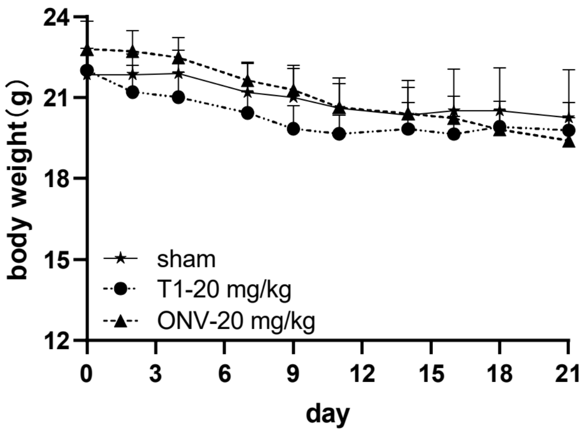


FIG. 12

## INTERNATIONAL SEARCH REPORT

International application No.
**PCT/CN2024/097937**

**A. CLASSIFICATION OF SUBJECT MATTER**

C07D 487/04(2006.01)i; A61K 31/517(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: C07D; A61K; A61P

Documentation searched other than minimum documentation to the extent that such documents are included in the fields searched

Electronic data base consulted during the international search (name of data base and, where practicable, search terms used)

CNTXT, ENTXTC, DWPI, CNKI, STN(CAPLUS, REGISTRY): 吡唑并喹唑啉, PLK1激酶抑制剂, 结晶, 晶体, 晶型, 晶形, 晶, pyrazolo-quinazoline, PLK1 kinase inhibitor, crystal, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| A | CN 103626777 A (NERVIANO MEDICAL SCIENCES S.R.L.) 12 March 2014 (2014-03-12) claims 1-3 | 1-15 |
| A | CN 102470136 A (NERVIANO MEDICAL SCIENCES S.R.L.) 23 May 2012 (2012-05-23) claims 1-12 | 1-15 |
| A | CN 102079746 A (NERVIANO MEDICAL SCIENCES S.R.L.) 01 June 2011 (2011-06-01) claims 1-32, and description, paragraphs [1212-1216] | 1-15 |
| A | WO 2019029663 A1 (SHENGKE PHARMACEUTICALS (JIANGSU) LTD.) 14 February 2019 (2019-02-14) claims 1-10 | 1-15 |
| A | CN 103298816 A (NERVIANO MEDICAL SCIENCES S.R.L.) 11 September 2013 (2013-09-11) claims 1-25, and see description, paragraphs [0583-0590] | 1-15 |
| PX | WO 2023104178 A1 (SHANDONG LUYE PHARMACEUTICAL CO., LTD.) 15 June 2023 (2023-06-15) embodiment 1, and claims 10-14 | 1-15 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 August 2024** | **16 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/097937**

**C. DOCUMENTS CONSIDERED TO BE RELEVANT**

| Category* | Citation of document, with indication, where appropriate, of the relevant passages | Relevant to claim No. |
|---|---|---|
| PX | LU, Jing et al. "Design, Synthesis, and Biological Evaluation of Novel Molecules as Potent Inhibitors of PLK1" *Bioorganic Chemistry,* Vol. 139, 13 July 2023 (2023-07-13), 106711 page 8, table 4 | 1-15 |

Form PCT/ISA/210 (second sheet) (July 2022)

**INTERNATIONAL SEARCH REPORT**
**Information on patent family members**

International application No.
**PCT/CN2024/097937**

| Patent document cited in search report | Publication date (day/month/year) | Patent family member(s) | Publication date (day/month/year) |
|---|---|---|---|
| CN 103626777 A | 12 March 2014 | WO 2011012534 A1 | 03 February 2011 |
| | | TW 201109334 A | 16 March 2011 |
| | | CN 102470136 A | 23 May 2012 |
| | | EP 2459196 A1 | 06 June 2012 |
| | | US 2012157468 A1 | 21 June 2012 |
| | | HK 1166015 A0 | 19 October 2012 |
| | | JP 2013500299 A | 07 January 2013 |
| | | CN 102470136 B | 25 December 2013 |
| | | US 8648078 B2 | 11 February 2014 |
| | | HK 1166015 A1 | 15 August 2014 |
| | | HK 1195773 A0 | 21 November 2014 |
| | | EP 2459196 B1 | 09 September 2015 |
| | | CN 103626777 B | 21 October 2015 |
| | | ES 2553114 T3 | 04 December 2015 |
| | | JP 5851990 B2 | 03 February 2016 |
| | | HK 1195773 A1 | 14 October 2016 |
| CN 102470136 A | 23 May 2012 | JP 2013500299 A | 07 January 2013 |
| | | JP 5851990 B2 | 03 February 2016 |
| | | TW 201109334 A | 16 March 2011 |
| | | AR 077422 A1 | 24 August 2011 |
| | | WO 2011012534 A1 | 03 February 2011 |
| | | US 2012157468 A1 | 21 June 2012 |
| | | US 8648078 B2 | 11 February 2014 |
| | | EP 2459196 A1 | 06 June 2012 |
| | | EP 2459196 B1 | 09 September 2015 |
| | | ES 2553114 T3 | 04 December 2015 |
| CN 102079746 A | 01 June 2011 | US 2017050972 A1 | 23 February 2017 |
| | | US 9637497 B2 | 02 May 2017 |
| | | RS 52899 B | 28 February 2014 |
| | | US 2015158876 A1 | 11 June 2015 |
| | | US 9464090 B2 | 11 October 2016 |
| | | BR 122019010200 B1 | 15 December 2020 |
| | | BR 122019010200 B8 | 27 July 2021 |
| | | US 2007185143 A1 | 09 August 2007 |
| | | US 7482354 B2 | 27 January 2009 |
| | | MY 142019 A | 16 August 2010 |
| | | HRP 20050967 A2 | 31 May 2007 |
| | | HRP 20050967 B1 | 28 March 2014 |
| | | HRP 20050967 B8 | 25 April 2014 |
| | | US 2013338148 A1 | 19 December 2013 |
| | | US 8981089 B2 | 17 March 2015 |
| | | GEP 20094664 B | 10 April 2009 |
| | | CR 8102 A | 09 August 2006 |
| | | US 2019194214 A1 | 27 June 2019 |
| | | ECSP 056194 A | 19 April 2006 |
| | | SI 1636236 T1 | 31 January 2014 |
| | | TW 200505924 A | 16 February 2005 |
| | | TWI 349672 B | 01 October 2011 |
| | | EP 1636236 A1 | 22 March 2006 |
| | | EP 1636236 B1 | 09 October 2013 |

Form PCT/ISA/210 (patent family annex) (July 2022)

**INTERNATIONAL SEARCH REPORT**
**Information on patent family members**

International application No.
**PCT/CN2024/097937**

| Patent document cited in search report | Publication date (day/month/year) | Patent family member(s) | Publication date (day/month/year) |
|---|---|---|---|
| | | OA 13170 A | 13 December 2006 |
| | | JP 2007502851 A | 15 February 2007 |
| | | JP 5043432 B2 | 10 October 2012 |
| | | AU 2004240772 A1 | 02 December 2004 |
| | | AU 2004240772 B2 | 28 April 2011 |
| | | BRPI 0410563 A | 20 June 2006 |
| | | BRPI 0410563 B1 | 01 December 2020 |
| | | BRPI 0410563 B8 | 25 May 2021 |
| | | US 2021300935 A1 | 30 September 2021 |
| | | TNSN 05298 A1 | 10 July 2007 |
| | | MXPA 05012573 A | 22 February 2006 |
| | | CY 1114708 T1 | 05 October 2016 |
| | | NZ 543661 A | 31 May 2009 |
| | | MEP 25908 A | 10 June 2010 |
| | | ME 00142 B | 10 October 2010 |
| | | WO 2004104007 A1 | 02 December 2004 |
| | | RS 20050944 A | 05 June 2008 |
| | | ES 2436524 T3 | 02 January 2014 |
| | | UA 80763 C2 | 25 October 2007 |
| | | AR 044543 A1 | 21 September 2005 |
| | | IS 8132 A | 18 November 2005 |
| | | IS 2939 B | 15 March 2016 |
| | | PT 1636236 E | 16 December 2013 |
| | | NO 20055496 D0 | 21 November 2005 |
| | | NO 20055496 L | 14 February 2006 |
| | | NO 334992 B1 | 18 August 2014 |
| | | ZA 200509486 B | 25 September 2008 |
| | | EA 200501849 A1 | 30 June 2006 |
| | | EA 010904 B1 | 30 December 2008 |
| | | IL 172046 A | 30 March 2017 |
| | | DK 1636236 T3 | 09 December 2013 |
| | | US 2017190714 A1 | 06 July 2017 |
| | | US 10280176 B2 | 07 May 2019 |
| | | PL 1636236 T3 | 28 February 2014 |
| | | CO 5721006 A2 | 31 January 2007 |
| | | AR 102722 A2 | 22 March 2017 |
| | | CA 2526578 A1 | 02 December 2004 |
| | | CA 2526578 C | 24 January 2012 |
| | | AP 200503452 A0 | 31 December 2005 |
| | | AP 2005003452 A0 | 30 October 2009 |
| | | US 2009124605 A1 | 14 May 2009 |
| | | US 8541429 B2 | 24 September 2013 |
| | | KR 20060015294 A | 16 February 2006 |
| | | KR 101084871 B1 | 21 November 2011 |
| WO 2019029663 A1 | 14 February 2019 | US 2021147424 A1 | 20 May 2021 |
| | | US 11319323 B2 | 03 May 2022 |
| | | US 2022227775 A1 | 21 July 2022 |
| | | US 11993604 B2 | 14 February 2019 |
| | | EP 3666774 A1 | 17 June 2020 |
| | | EP 3666774 A4 | 11 March 2021 |

Form PCT/ISA/210 (patent family annex) (July 2022)

**INTERNATIONAL SEARCH REPORT**
**Information on patent family members**

International application No.
**PCT/CN2024/097937**

| Patent document cited in search report | | | Publication date (day/month/year) | Patent family member(s) | | | Publication date (day/month/year) |
|---|---|---|---|---|---|---|---|
| | | | | EP | 3666774 | B1 | 11 May 2022 |
| | | | | JP | 2020530491 | A | 22 October 2020 |
| | | | | JP | 7233743 | B2 | 07 March 2023 |
| | | | | KR | 20200038286 | A | 10 April 2020 |
| | | | | KR | 20220088518 | A | 27 June 2022 |
| | | | | KR | 102464677 | B1 | 10 November 2022 |
| | | | | ES | 2923415 | T3 | 27 September 2022 |
| CN | 103298816 | A | 11 September 2013 | KR | 20140027902 | A | 07 March 2014 |
| | | | | KR | 101913441 | B1 | 30 October 2018 |
| | | | | BR | 112013008527 | A2 | 12 July 2016 |
| | | | | WO | 2012080990 | A1 | 21 June 2012 |
| | | | | JP | 2014503531 | A | 13 February 2014 |
| | | | | JP | 5980225 | B2 | 31 August 2016 |
| | | | | RU | 2013109776 | A | 27 January 2015 |
| | | | | RU | 2652638 | C2 | 28 April 2018 |
| | | | | EP | 2614065 | A1 | 17 July 2013 |
| | | | | EP | 2614065 | B1 | 19 April 2017 |
| | | | | MX | 2013003328 | A | 24 June 2013 |
| | | | | MX | 342873 | B | 17 October 2016 |
| | | | | US | 2012190678 | A1 | 26 July 2012 |
| | | | | US | 8541576 | B2 | 24 September 2013 |
| | | | | CA | 2812223 | A1 | 21 June 2012 |
| | | | | CA | 2812223 | C | 12 March 2019 |
| WO | 2023104178 | A1 | 15 June 2023 | CN | 118382626 | A | 23 July 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022137824 W